# FASCICULE DE BREVET EUROPEEN

(11) **EP 0 950 047 B1**
(45) Date de publication et mention de la délivrance du brevet: **15.10.2003**
(21) Numéro de dépôt: 97951307.4
(22) Date de dépôt: 11.12.1997
(51) Int. Cl.: C07D 209/96, A61K 31/40, C07D 401/12, C07D 491/10

(54) **DERIVES D'INDOLIN-2-ONE, PROCEDE POUR LEUR PREPARATION ET COMPOSITIONS PHARMACEUTIQUES LES CONTENANT**
INDOLIN-2-ONE DERIVATE, VERFAHREN ZU IHRER HERSTELLUNG UND DIESEENTHALTENDE ARZNEIMITTEL
INDOLIN-2-ONE DERIVATIVES, METHOD FOR PREPARING THEM AND PHARMACEUTICAL COMPOSITIONS CONTAINING THEM

(30) Priorité: 13.12.1996 FR 9615384
(43) Date de publication de la demande: 20.10.1999
(73) Titulaire: SANOFI-SYNTHELABO, 75013 Paris (FR)
(72) Inventeur: FOULON, Loic, F-31120 Pinsaguel (FR); SERRADEIL-LE GAL, Claudine, F-31750 Escalquens (FR); VALETTE, Gérard, F-31120 Lacroix-Falgarde (FR)
(74) Mandataire: Gillard, Marie-Louise
(86) Numéro de dépôt international: FR9702270
(87) Numéro de publication internationale: WO98025901

(56) Documents cités:
- EP-A- 0 636 608
- EP-A- 0 636 609
- WO-A-97/15556

## Description

La présente invention a pour objet de nouveaux dérivés d'indolin-2-one, un procédé pour leur préparation et les compositions pharmaceutiques les contenant. Ces nouveaux dérivés sont pourvus d'affinité pour les récepteurs de la vasopressine et/ou de l'ocytocine et peuvent donc constituer des principes actifs de compositions pharmaceutiques.

La vasopressine est une hormone connue en particulier pour son effet antidiurétique et son effet dans la régulation de la pression artérielle. Elle stimule plusieurs types de récepteurs : V₁ (V₁ₐ, V_{1b} ou V₃), V₂. Ces récepteurs sont localisés dans le foie, les vaisseaux (coronaires, rénaux, cérébraux), les plaquettes, le rein, l'utérus, le pancréas, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus ; ils se trouvent essentiellement sur les cellules myoépithéliales de la glande mammaire, dans le système nerveux central, le rein, les vaisseaux et l'adipocyte. La localisation des différents récepteurs est décrite dans : Jard S. *et al.,* "Vasopressin and oxytocin receptors : an overview, in Progress" dans Endocrinology., Imura H. and Shizume K. ed., Experta Medica, Amsterdam, 1988, 1183-1188, ainsi que dans les articles suivants : Presse Médicale, 1987, 16 (10), 481-485, J. Lab. Clin. Med., 1989, *114* (6), 617-632 et Pharmacol. Rev., 1991 *43* (1), 73₋108. La vasopressine exerce ainsi des effets hormonaux, cardiovasculaires, hépatiques, rénaux, antidiurétiques, agrégants et des effets sur les systèmes nerveux central et périphérique, sur les sphères utérine et intestinale et sur le système oculaire et pulmonaire. L'ocytocine intervient dans la parturition, la. lactation, le comportement sexuel et la régulation du métabolisme des graisses.

Les antagonistes des récepteurs V₂ de la vasopressine (appelés également "AVP-2-antagonistes" ou "antagonistes V₂") sont préconisables comme puissants aquarétiques qui interviennent spécifiquement sur la réabsorption rénale de l'eau sans entraîner de fuites électrolytiques (Na⁺, K⁺) comme le font les diurétiques classiquement utilisés en clinique, tels que le furosemide ou l'hydrochlorothiazide.

Ces derniers entraînent après un traitement prolongé des hypokaliémies et hyponatrémies.

Le premier antagoniste des récepteurs V₂ de l'arginine-vasopressine (ci-après dénommée AVP), l'OPC-31260, est actuellement en cours de développement clinique. La comparaison des effets de l'OPC-31260 aux diurétiques classiques, tel que le furosemide, démontre que, tant chez l'animal (Yoshitaka Y. *et al*., Br. J. Pharmacol., 1992, *105,* 787-791) que chez l'homme (Akihiro O. *et al*., J. Clin. Invest., 1993, 92, 2653-2659, Akihiro O. *et al*.. J*.* Pharmacol. Exp. Ther., 1995, 272, 546-551) un tel composé favorise sélectivement la diurèse aqueuse et n'affecte pas, ou très peu aux fortes doses, l'excrétion des ions.

Des dérivés d'indolin-2-one ont été décrits dans la littérature. A titre d'exemple, on peut citer le brevet ZA 830952 qui décrit des dérivés utiles comme antihypertenseurs qui inhibent l'enzyme de conversion, le brevet FR 1509373 qui décrit des composés diurétiques pourvus d'un effet sur l'excrétion du potassium.

Plusieurs demandes de brevet ou brevets décrivent également des séries de composés non peptidiques possédant une affinité pour les récepteurs de la vasopressine et/ou de l'ocytocine. C'est le cas par exemple de EP 382185 qui décrit des dérivés de carbostyryle qui sont des antagonistes de la vasopressine utiles comme vasodilatateurs, hypotenseurs, diurétiques et antiagrégants plaquettaires ; de EP 444945 qui décrit des dérivés de spiropipéridine utiles notamment dans la dysménorrhée ; de EP 514667 qui décrit des dérivés de benzazépine utiles notamment dans les troubles de la fonction rénale, dans l'hyponatrémie, le diabète ou encore dans le traitement et la prophylaxie de l'hypertension et'dans l'inhibition de l'agrégation plaquettaire ; ou encore de JP 03127732 qui décrit des dérivés d'indoles comme antagonistes de la vasopressine.

Des dérivés de benzyle ou sulfonylindoline et d'indole ont également été décrits comme antagonistes de la vasopressine et ou de l'ocytocine. A cet effet, on peut citer les documents EP 469984, EP 526348, EP 636608, EP 636609, WO 93/15051 et WO 95/18105.

Il a été maintenant trouvé que ces indolinones présentent une excellente affinité vis-à-vis des récepteurs de la vasopressine et/ou de l'ocytocine. Ces nouvelles indolin-2-ones sont des antagonistes puissants des récepteurs V₂ de la vasopressine et éventuellement des récepteurs de l'ocytocine. De plus, compte tenu de leur structure et en particulier de la présence de diverses fonctions polaires, notamment des fonctions salifiables, ces molécules possèdent une bonne dispersibilité et/ou solubilité dans l'eau qui leur confère une activité pharmacologique améliorée et une excellente biodisponibilité et permettent aussi la préparation aisée de formes galéniques injectables.

Ainsi, selon l'un de ses aspects, la présente invention concerne de nouvelles indolin-2-ones répondant à la formule : 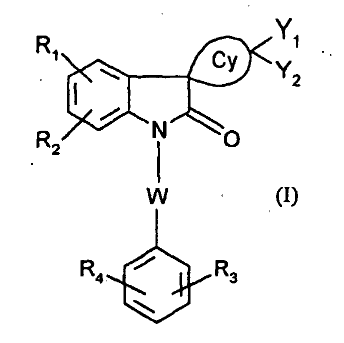 dans laquelle :
- R₁ est en position 5 de l'indolin-2-one et représente un hydrogène ; un hydroxyle; un halogène ; un (C₁-C₇)alkyle ; un (C₁-C₇)polyfluoroalkyle ; un (C₁-C₇)alcoxy ; un (C₁-C₇)alkylthio ; un (C₁-C₇)polyfluoroalcoxy ; un (C₃-C₇)cycloalkyloxy ; un (C₃-C₇) cycloalkylthio ; un cycloalkylméthoxy ou un cycloalkylméthylthio dans lesquels le cycloalkyle est en C₃-C₇ ; un phénoxy; un benzyloxy ; un nitro ; un cyano ;
- R₂ représente l'hydrogène ;
- R₃ et R₄ indépendamment l'un de l'autre substituent une ou plusieurs fois le groupe phényle et représentent chacun indépendamment un hydrogène ; un halogène ; un (C₁-C₇)alkyle ; un (C₂-C₇)alcényle ; un (C₁-C₇)polyhalogénoalkyle ; un phényle ou un benzyle ; un cyano ; un nitro ; un groupe -NR₅R₆ ; un hydroxyamino ; un hydroxyle ; un groupe OR₇ ; un groupe SR₇ ; un groupe -COOR₈ : un groupe -CONR₉R₁₀ ; un groupe -CSNR₉R₁₀, l'un au moins des radicaux R₃ et R₄ étant différent de l'hydrogène ;
- R₅ et R₆ représentent chacun indépendamment un hydrogène ; un (C₁-C₇)alkyle ; un (C₂-C₇)alcényle : un phényle ; un benzyle ; un (C₁-C₇)alkylcarbonyle ; un (C₁-C₇)alkylthiocarbonyle ; un (C₃-C₇)cycloalkylcarbonyle ; un (C₃-C₇)cycloalkylthiocarbonyle ; un benzoyle; un thiénylcarbonyle ; un furylcarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀ ou bien R₅ et R₆ constituent avec l'atome d'azote auquel ils sont liés un groupe hétérocyclique choisi parmi les groupes pyrrolidine, pyrroline, pyrrole, indoline, indole, pipéridine ;
   ou bien R₅ constitue avec l'atome d'azote auquel il est lié et l'atome de carbone adjacent du phényle, un hétérocycle choisi parmi indole, indoline et tétrahydroquinoline et alors R₆ représente un hydrogène ; un (C₁-C₇)alkyle; un benzyle ; un (C₁-C₇)alkylcarbonyle ; un (C₁-C₇)thiocarbonyle ; un (C₃-C₇)cycloalkylcarbonyle : un (C₃-C₇)cycloalkylthiocarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀ ;
- R₇ représente un (C₁-C₇)alkyle ; un (C₂-C₇)alcényle ; un phényle ; un benzyle ; un (C₃-C₇)cycloalkyle ; un (C₁-C₇)polyfluoroalkyle ; un formyle ; un (C₁-C₇) alkylcarbonyle ; un benzoyle; un benzylcarbonyle ;
- R₈ représente un hydrogène, un (C₁-C₇)alkyle ; un phényle ; un benzyle ;
- R₉ et R₁₀ représentent chacun indépendamment l'hydrogène ; un (C₁-C₇)alkyle ; un (C₁-C₇)polyfluoroalkyle ; un (C₂-C₇)alcényle ; un (C₃-C₇)cycloalkyle éventuellement substitué par un groupe hydroxy(C₁-C₄)alkyle ; un pyridyle ; un phényle ; un thiényle ; un furyle ; ou bien R₉ et R₁₀ constituent avec l'atome d'azote auquel ils sont liés un groupe hétérocyclique choisi parmi les groupes pyrrolidine, pipéridine ou pipérazine non substitués ou substitués par un ou plusieurs groupe(s) (C₁-C₄)alkyle(s) ; ou un (C₄-C₇) azacycloalkyle ;
- W représente un groupe -SO₂- ;
- Cy constitue, avec le carbone auquel il est lié, un cycle hydrocarboné non aromatique en C₅-C₁₂, saturé ou insaturé, éventuellement condensé ou substitué par un ou plusieurs groupe(s) (C₁-C₇)alkyle(s), lesdits groupes pouvant substituer une ou plusieurs fois le même atome de carbone, ou par un spirocycloalkyle en C₃-C₆;
- Y₁ et Y₂ substituent le même atome de carbone de Cy et
- Y₁ représente
   (i) - un groupe (C₀-C₄)alkylène -T-Z ,
   (ii) - un groupe (C₀-C₃)alkylène -NR₁₆-T-Z dans lequel R₁₆ représente un atome d'hydrogène ou un (C₁-C₃)alkyle;
- Y₂ représente un atome d'hydrogène ou un groupe hydroxyle ou bien forme avec Y₁ un groupe (C₁-C₄)alkylidène-T-Z ;
- T représente un (C₁-C₄)alkylène ou une liaison directe ;
- Z représente un groupe -NR₁₁R₁₂ ; un groupe -COOR₁₁ ; un groupe -CONR₁₁R₁₂; étant entendu que
   lorsque Y₁ est tel que défini dans le cas (ii) et lorsque T représente un groupe méthylène ou une liaison directe, alors Z ne peut pas être -NR₁₁R₁₂ ;
- R₁₁ et R₁₂ représentent chacun indépendamment l'hydrogène ou un (C₁-C₇)alkyle ; lesdits groupes pouvant éventuellement être mono ou polysubstitués par R₁₃;
   ou bien R₁₁ et R₁₂ constituent éventuellement avec l'atome d'azote auquel ils sont liés un hétérocycle choisi parmi les hétérocycles pipéridine et morpholine, éventuellement mono ou polysubstitué par R₁₃;
- R₁₃ représente un benzyloxy; un groupe NR₁₄R₁₅ dans lequel R₁₄ et R₁₅ représentent chacun indépendamment l'hydrogène ou un (C₁-C₄)alkyle;
ainsi que leurs sels, solvates ou hydrates.

Parmi les composés ci-dessus, on préfère tout particulièrement ceux dans lesquels Y₁ est :
- soit le groupe (C₀-C₄)alkylène-T-Z dans lequel Z est le groupe -NR₁₁R₁₂;
- CONR₁₁R₁₂; COOR₁₁ et T est une liaison directe ou un groupe (C₁-C₄)alkylène, de préférence une liaison directe ;
- soit le groupe (C₀-C₃)alkylène-NR₁₆-T-Z dans lequel T est un (C₁-C₄)alkylène et Z est -NR₁₁R₁₂ ;

Selon la présente invention, par "(C₁-C₇)alkyle, (C₁-C₇)alkylène, (C₁-C₇)alkylidène" on entend un alkyle, un alkylène ou un alkylidène droit ou ramifié ayant 1 à 7 atomes de carbone.

Les cycles hydrocarbonés non aromatiques en C₅-C₁₂ comprennent les radicaux mono- ou polycycliques, condensés ou pontés, saturés ou insaturés, éventuellement terpéniques. Ces radicaux sont éventuellement mono- ou polysubstitués par un (C₁-C₄)alkyle. Les radicaux monocycliques incluent les cycloalkyles par exemple les cyclopentyles, cyclohexyles, cycloheptyles, cyclooctyles, cyclododécyles. Les radicaux polycycliques incluent par exemple le norbomane, l'adamantane, l'hexahydroindane, le norbomène, le dihydrophénalène, le bicyclo [2.2.1]heptane. le bicyclo [3.3.1]nonane; le tricyclo [5.2.1.0^{2,6}]decane.

Selon la présente invention, par halogène on entend un atome choisi parmi le fluor, le chlore, le brome ou l'iode, de préférence le fluor ou le chlore.

Lorsqu'un composé selon l'invention présente un ou des carbones asymétriques, les isomères optiques de ce composé font partie intégrante de l'invention.

Lorsqu'un composé selon l'invention présente une stéréoisomérie par exemple de type axial-équatorial ou Z-E, l'invention comprend tous les stéréoisomères de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Les sels des composés de formule (I) comprennent également des sels avec des bases organiques ou minérales, par exemple les sels des métaux alcalins ou alcalinoterreux, comme les sels de sodium, de potassium, de calcium, les sels de sodium et de potassium étant préférés, ou avec une amine, telle que le trométamol, du bien les sels d'arginine, de lysine, ou de toute aminé physiologiquement acceptable.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus.

Par groupe protecteur temporaire des amines, alcools, phénols, thiols ou des acides carboxyliques on entend les groupes protecteurs tels que ceux décrits dans Protective Groups in Organic Synthesis, Greene T.W. et Wuts P.G.M., ed. John Wiley & Sons, 1991 et dans Protective Groups, Kocienski P. J., 1994, Georg Thieme Verlag.

On peut citer par exemple des groupements protecteurs temporaires des amines: benzyles, carbamates (tels que *tert*-butyloxycarbonyle clivables en milieu acide, benzyloxycarbonyle clivables par hydrogénolyse), des acides carboxyliques (esters d'alkyle tels méthyle ou éthyle, *tert*-butyle hydrolysables en milieu basiques ou acides, benzyliques hydrogènolysables), des alcools ou des phénols tels que des éthers de tétrahydropyranyle, méthyloxyméthyle ou méthyléthoxyméthyle, *tert*-butyle et benzyle et se référer aux méthodes générales bien connues décrites dans Protective Groups, cité ci-dessus.

Les groupes protecteurs permanents sont ceux qui sont stables dans les conditions de clivage citées ci-dessus et qui sont susceptibles d'être présents dans les produits finaux. De tels groupes 0-protecteurs ou N-protecteurs sont constitués par les groupes (C₁-C₇)alkyle, phényle. Les groupes N-protecteurs permanents incluant également les groupes (C₁-C₅)alcanoyles et les groupes aroyles, tel que le benzoyle.

Les composés (I) peuvent comporter des groupes précurseurs d'autres fonctions qui sont générées ultérieurement en une ou plusieurs autres étapes.

On préférera selon la présente invention les groupements protecteurs temporaires clivables en milieu acide, ou en milieu neutre par hydrogénolyse.

Les composés de formule (I) dans lesquels les diverses fonctions polaires, notamment les fonctions salifiables qui améliorent la solubilité et/ou la dispersibilité dans l'eau sont de préférence portés par le groupe Y₁.

La présente invention a également pour objet un procédé pour la préparation des composés de formule (I).

Les composés selon l'invention peuvent être préparés selon le SCHEMA 1 ci-après:

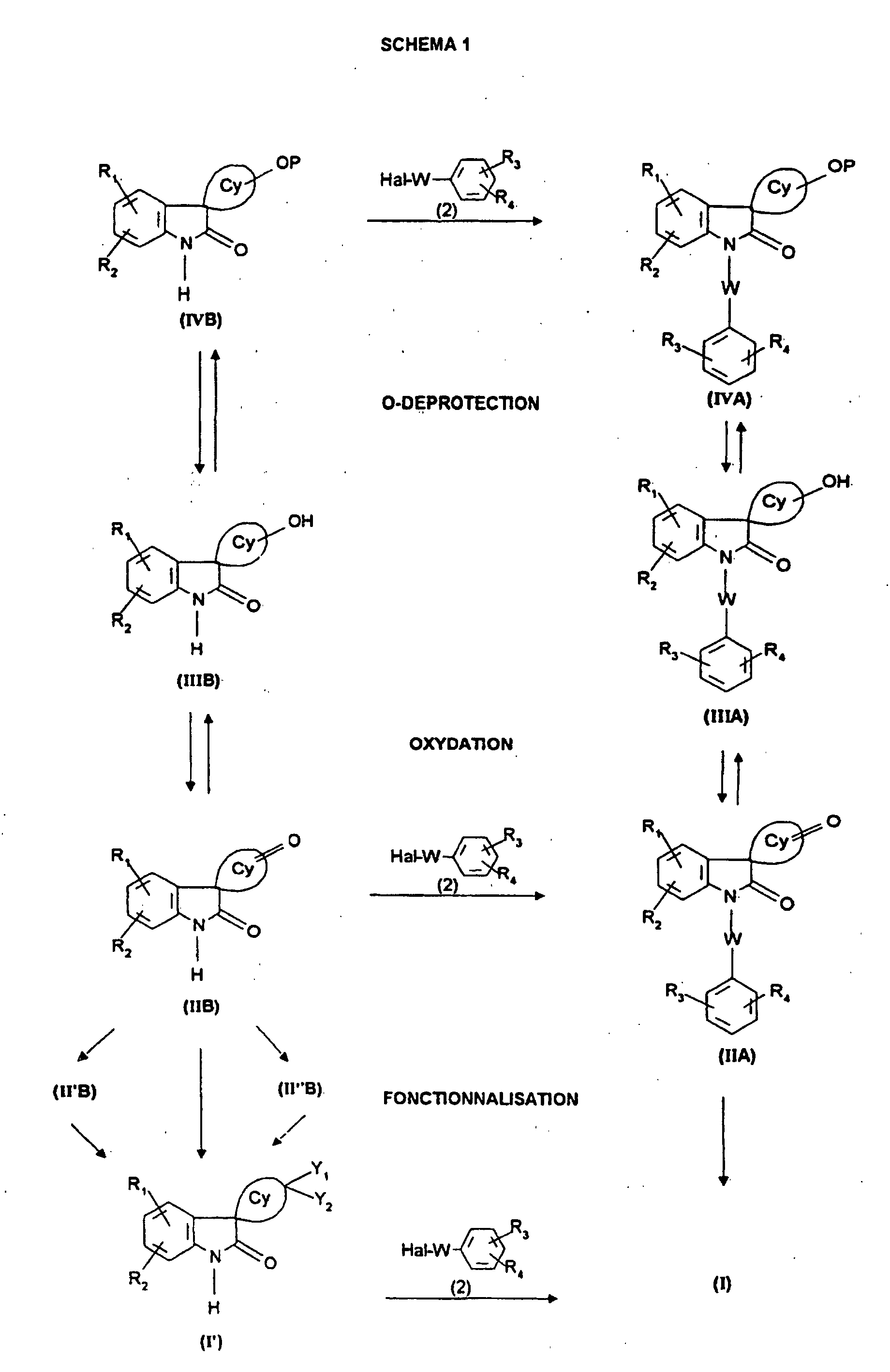

Dans le schéma 1 ci-dessus, les composés (II'B) sont les composés répondant à la formule ci-après : 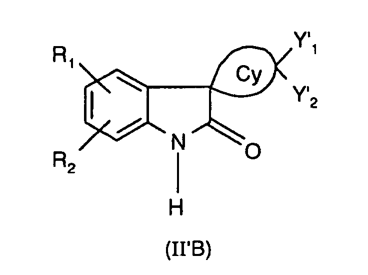 dans lesquels Y'₁ et Y'₂ représentent Y₁ et/ou Y₂ ou un précurseur de Y₁ et/ou Y₂.

De préférence, les groupes Y'₁ sont l'un des groupes ci-après :
(i) un groupe (C₀-C₄)alkylène-X
(ii) un groupe (C₀-C₃)alkylène-X
ou bien forme avec Y'₂ un groupe (C₁-C₄)alkylidene-X;
dans lesquels X représente un hydroxyle, un (C₁-C₃)alcoxy, un halogénure, un ester d'acide sulfonique tel qu'un tosyloxy ou un mésyloxy, un cyano, un azido, un nitro ou constitue avec l'alkylène ou l'alkylidène auquel il est lié, un aldéhyde ou une cétone.

Le composé (II"B) répond à la formule : 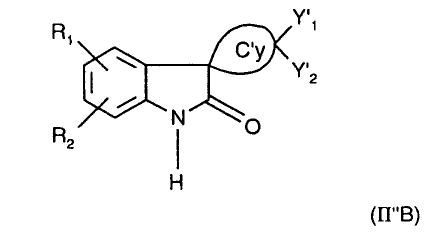 dans laquelle C'y constitue avec le carbone auquel il est lié un cycle hydrocarboné insaturé.

La présente invention a également pour objet un procédé pour la préparation des composés de formule (I) caractérisé en ce que :
1) on fait réagir un composé (I') de formule : 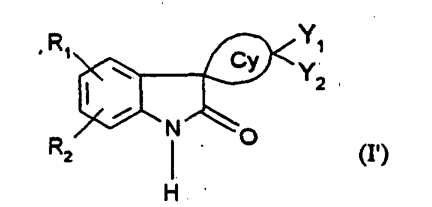 dans lequel R₁,. R₂, Cy, Y₁ et Y₂ sont tels que définis pour (I) avec un composé de formule: 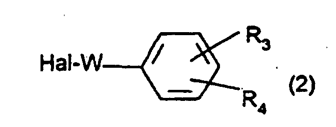 dans lequel W, R₃ et R₄ sont tels que définis pour (I) et Hal représente un atome d'halogène, en présence d'un hydrure métallique comme par exemple l'hydrure de sodium ou d'un alcoolate alcalin comme par exemple le tert-butylate de potassium à des températures comprises entre -40° et 25°C, dans un solvant anhydre tel que le tétrahydrofurane ;
2) ou bien on peut faire réagir de nombreux réactifs nucléophiles tels que des thiols, amines, ou carbanions sur les dérivés carbonylés. (IIA) selon les réactions très générales bien connues de l'homme de l'art pour obtenir directement les composés (I) selon l'invention.

Dans la présente description, on nomme l'étape transformant (IIA) en (I) "étape de fonctionnalisation" qui est de même nature que celle qui transforme les composés (IIB) en composés (I'), (II'B) ou (II"B). Ces transformations seront donc décrites ci-après ensemble. L'homme de l'art saura choisir entre les composés (IIA) et (IIB) susceptibles d'être soumis à l'étape de fonctionnalisation en fonction des réactions mises en oeuvre. Par exemple, les réactions de réduction par des réducteurs, tels que le borohydrure de sodium ou les hydrolyses en milieu basique seront plutôt mises en oeuvre à partir des composés (IIB) ; les réactions mettant en jeu des carbanions seront préférées sur les composés (IIA) ou aménagées pour tenir compte de la fonction lactame des composés (IIB), par exemple en la protégeant.

Très généralement, les composés de formule (I) ou (I') et les intermédiaires de formule (II'B) peuvent être préparés par des réactions d'homologation, c'est-à-dire de couplage carbone-carbone classiques à partir des dérivés carbonylés (IIA) et (IIB) respectivement. On peut consulter les méthodes décrites dans Synthesis, 1979, 633-665 qui sont applicables aux fins de l'invention.

Les composés (I) ou (I'),ou (II'B) dans lesquels Y₁+Y₂ forment un groupe (C₁-C₄)alkylidène-T-Z sont avantageusement préparés par la réaction sur les dérivés carbonylés (IIA) ou (IIB) de dérivés du phosphore tels que les ylures de phosphonium, les anions des oxydes de phosphines ou des phosphonates ; ces réactions sont connues sous le nom de réactions de Wittig, Wittig-Horner ou Horner-Wadworth-Emmons et sont largement décrites et exemplifiées dans la littérature. On peut consulter Org. Reactions, 1965, 14, 270 ; Chem. Organophophorus Compounds; 1994, 185 ; Org. Reactions, 1977, 25, 73 ; Chem. Rev., 1974 et 1989, 74 et 89, 87 et 863 respectivement.

Les composés (II'B) comportant des fonctions alcools, aldéhydes ou cétones et les composés (I) ou (I') comportant des fonctions acides carboxyliques ou esters peuvent être interconvertis par les procédés classiques d'oxydation ou de réduction bien connus de l'homme du métier.

Les composés (II'B) dans lesquels Y'₁ représente un groupe cyano sont avantageusement préparés par réaction des composés carbonylés (IIA) ou (IIB) avec le tosylméthylisonitrile dans les conditions décrites dans J. Org. Chem., 1977, *42*, 3114-3118.

On peut préparer les composés (I) ou (I') dans lesquels Y, représente un groupe alkylène (C₁-C₃) substitué par un groupe -NR₁₆-T-Z par les réactions classiques mettant en jeu les composés (II'B) dans lesquels Y'₁, représente le même groupe alkylène substitué par un groupe X, X étant défini comme un groupe nucléofuge tel qu'un halogène de préférence brome, chlore ou iode, ou un dérivé d'acide sulfonique, tel que tosyloxy, mésyloxy avec une amine HNR₁₆-T-Z dans des solvants polaires, tels que le diméthylformamide, le tétrahydrofurane ou l'acétonitrile à des températures comprises entre 0°C et 120°C. X peut représenter également un groupe azido réductible en amino. Les composés (II'B) comprenant X définis ci-dessus sont en général préparés à partir des alcools correspondants. On peut citer par exemple des systèmes triphénylphosphine/tétrachlorure de carbone selon Angew. Chem. Int. Ed., 1975, *14*, 801 ou triphénylphosphine/C(Hal)₄ dans lequel Hal représente un halogène en présence de pyridine selon Carbohyd. Res., 1978, *61*, 511 ou par réaction avec un halogénure d'aryle- ou d'alkyle-sulfonyle en présence d'une base dans un solvant neutre. Les groupes X peuvent s'échanger : par exemple on peut transformer un groupe sulfonate en un halogénure, tel qu'un dérivé de l'iod par une réaction avec un iodure alcalin tel que l'iodure de sodium selon J. Chem. Soc., 1949, 326. Lorsque X représente un halogène on peut le transformer en hydroxy par substitution par un ion nitrate qui est ensuite réduit en présence d'un catalyseur métallique, tel que le palladium sur charbon selon la méthode décrite dans J. Med. Chem., 1995, *38*, 130-136.

On peut également faire appel. à des réactions d'aminatlon réductrice consistant à faire réagir une amine sur un dérivé carbonyié (IIA), (IIB)ou (II'B) en milieu acide en présence d'un agent réducteur. L'agent réducteur peut être l'hydrogène en présence d'un catalyseur métallique tel que le palladium, le nickel de Raney (on peut consulter M. Freifelder dans "Practical Hydrogenations in Organic Synthesis, Procedures and Commentary", John Wiley & Sons, New York, 1978, chapitre 10) ou des hydrures tels que BH₃, les borohydrures alcalins ou ses dérivés tels que le cyanoborohydrure de sodium en présence d'acide acétique (on peut consulter Org. Prep. Proc. Int., 1985, 17, 317) et plus particulièrement le triacétoxyborohydrure de sodium dans les conditions décrites dans Tetrah. Lett., 1990, 5595 ou J. Org. Chem., 1996, *61*, 3849-3862.

On peut également préparer les amines par les réductions bien connues des dérivés (II'B) dans lesquels X représente un nitro, azido ou cyano, en présence par exemple d'hydrogène et d'un catalyseur métallique tel que le palladium sur charbon ou l'oxyde de platine.

Les composés de formule (I) ou (I') peuvent comporter des fonctions amines ou acides qui peuvent être transformées en fonctions amides par réactions avec respectivement des dérivés d'acides ou d'amines pouvant comporter des carbones asymétriques. On se référera alors aux réactions de couplage non racémisantes bien connues de l'homme de l'art notamment dans la synthèse peptidique et on consultera Wunsch E., dans Methoden der Organischen Chemie (Synthese von peptiden), 1974, *15*,band 1+2, ,Thieme Verlag, Stuttgart ou Jones J.H., dans The Peptides, *1979, 1,* 65-104, Gross E., Meienhofer J., Academic Press, ou M. Bodansky, Principles of Peptide Synthesis et Peptide Chemistry, 1993, Springler Verlag.

Les ammoniums quaternaires, les dérivés N-oxydes, S-oxydes et les sulfones des composés (I) font partie de l'invention et sont préparés classiquement respectivement par réaction avec un halogénure d'alkyle, par oxydation avec de l'eau oxygénée ou un peracide, tel que l'acide paracétique ou métachloroperbenzoïque dans des solvants inertes.

Les composés (IIA) ou (IIB) sont préparés par l'oxydation des alcools secondaires correspondants (IIIA)ou (IIIB) selon les nombreuses méthodes bien connues de l'homme de l'art mettant par exemple en jeu des oxydants, tels que l'oxyde de chrome en milieu acétique ou des complexes de l'oxyde de chrome, tels que le chlorochromate de pyridinium dans des solvants inertes tels que l'acétate d'éthyte ou le dichlorométhane ou bien encore par hydrolyse des acétals (VA) ou (VB) (voir schéma 2 ci-après),

Les composés (IIIA) ou (IIIB) peuvent être préparés par hydrolyse des dérivés (IVA) ou (IVB) dans lesquels P est une fonction protectrice d'un fonction alcool par exemple un groupe méthoxyméthyle ou tétrahydropyranyle. L'hydrolyse s'effectue en milieu acide, par exemple, en présence d'acidé chlorhydrique dans un alcool tel que le méthanol, ou l'éthanol, un éther tel le tétrahydrofurane à des températures comprises entre -5°C et 70°C.

Les composés (IVA) et (IVB) sont décrits dans EP 636608 ou obtenus de maniére similaire.

Les composés (IIA) et (IIB) peuvent aussi être obtenus selon le SCHEMA 2 ci-après:

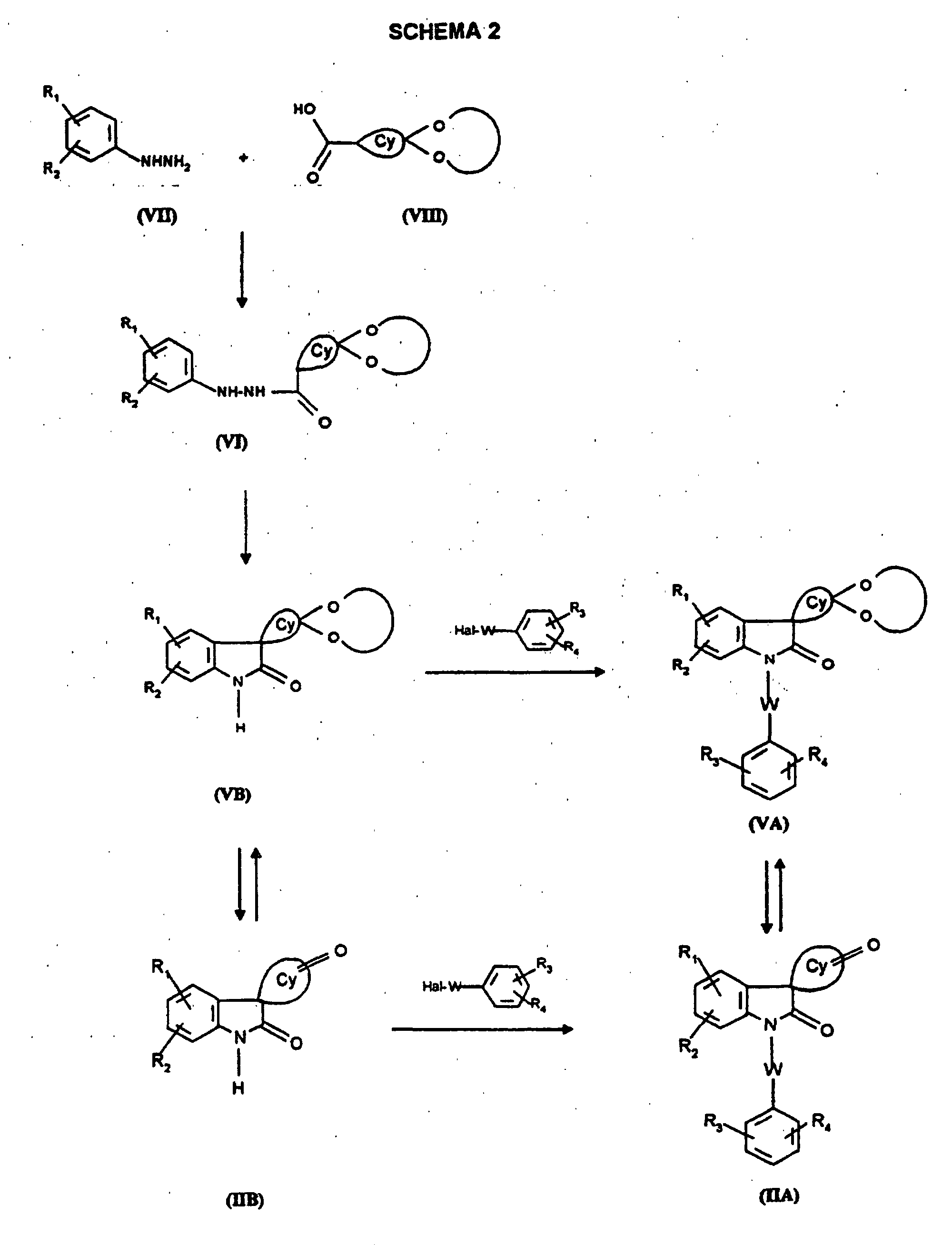

Les acétals (VA) ou (VB) peuvent être obtenus à partir des cétones correspondantes (IIA) ou (IIB) par réaction avec un diol tel que l'éthylèneglycol ou le propylène glycol en milieu déshydratant mais peuvent être plus avantageusement préparées directement à partir des hydrazides correspondants (VI) par une réaction de Brunner décrite par Moore R.F. *et al.,* J. Chem. Soc., 1951, 3475-3478, par exemple par chauffage dans des solvants tels que la quinoléine en présence d'un oxyde métallique ou alcalino-terreux comme l'oxyde de calcium. On peut également procéder par chauffage dans des solvants inertes, tels que la tétraline, le naphtalène ou le 1,2,3,4-tétraméthylbenzène selon la méthode décrite par Wolff J. et al., Tétrahedron, 1986, 42, (15), 4267-4272, à partir d'un sel. de lithium préparé au préalable dans un solvant inerte tel que le tétrahydrofurane à basse température.

Les phénylhydrazides (VI) peuvent être obtenus à partir d'une phénylhydrazine (VII), qui sont des composés connus ou préparés selon des méthodes connues, et de dérivés des acides carboxyliques (VIII}, tels que les esters, chlorures ou anhydrides mixtes obtenus par réaction d'un chloroformiate d'aikyle, de préférence d'isobutyle, en présence d'une base selon les méthodes classiques bien connues de l'homme de l'art.

Les acides (VIII) sont connus ou préparés selon des méthodes connues.

Les composés (IIA), (IVA) et (VA) peuvent étre préparés à partir des composés (IIB), (II'B), (IVB) et (VB) respectivement dans les conditions décrites pour la préparation des composés (I) à partir des composés (I').

Les réactifs de fonctionnalisation sont des composés connus ou préparés par des méthodes connues.

Les réactifs de formule (2): 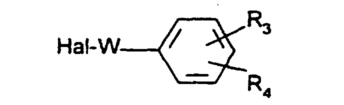 sont également préparés selon des méthodes connues. Notamment, les halogénures de benzènesulfonyle dans lesquels W = -SO₂- et R₃ et R₄ sont tels que définis précédemment pour (1), sont préparés par des méthodes connues. Ainsi par exemple, le chlorure de 4-diméthylaminobenzènesulfonyle est préparé selon Sukenik C.N. *et al.,* J. Am. Chem. Soc., 1977, *99*, 851-858. Plus généralement, les halogénures de benzènesulfonyle substitués par un groupe diméthylamino sont connus ou préparés par des méthodes connues ; le chlorure de 4-benzyloxybenzènesulfonyle est préparé selon la demande de brevet EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

On obtient les halogénures de benzènesulfonyle selon Col. Czechoslov. Chem. Commun., 1984, *49*, 1184, à partir des dérivés de l'aniline substitués par le même groupement, lesdits dérivés de l'aniline étant eux-mêmes obtenus à partir des dérivés nitrés correspondants.

L'halogénure de benzénesulfonyle (2) dans lequel le substituant en position 4 représente un groupe -NHCON(CH₂CH₃)₂ peut être préparé par action de l'acide chlorosulfonique sur la N',N'-diethyl-N-phénylurée, elle-même obtenue par réaction de l'aniline avec le chlorure de diéthylcarbamoyle.

Dans le cas où R₃ ou R₄ représentent un carbamoyle N-substitué, on peut condenser un composé (2) dans lequel R'₃ est un précurseur d'acide carboxylique, tel que N-benzylcarbamoyle, déprotèger le groupement protecteur par hydrogénolyse puis condenser avec l'amine désirée ou bien préparer directement (2) dans lequel R₃ à la valeur attendue. On opère généralement à partir des anilines correctement choisies, elles mêmes étant obtenues par réduction des dérivés nitrés correspondants. Les anilines sont diazotées dans les conditions classiques par l'acide nitreux et mises en réaction avec du SO₂ en présence de chlorure cuivrique selon J. Heterocyclic Chem., 1986, 23, 1253.

Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, et constituent de puissants ligands des récepteurs de la vasopressine et/ou de l'ocytocine.

L'affinité des composés selon l'invention pour les récepteurs V₁ de la vasopressine a été déterminée *in vitro* en utilisant la méthode décrite dans Lynch C.J. *et* al., *J.* Biol. Chem., 1985, *260* (5), 2844-2851. Cette méthode consiste à étudier le déplacement de la vasopressine tritiée fixée aux sites V₁ de membranes de foie de rat

De même, l'affinité des composés (I) selon l'invention pour les récepteurs de l'ocytocine a été déterminée *in vitro* par déplacement. d'un analogue radioiodé de l'ocytocine fixé aux récepteurs d'une préparation membranaire de glandes mammaires de rates en gestation, selon une technique proche de celle décrite par Elands J. *et al.,* dans Eur. J. Pharmacol., 1987, *147*,197-207. Certains composés selon l'invention inhibent la fixation d'un analogue de l'ocytocine radioiodée aux récepteurs des préparations membranaires. Leurs Cl₅₀ sont faibles, variant de 10⁻⁶ à 10⁻⁹M.

L'affinité des composés (I) selon l'invention pour les récepteurs V₂ a été mesurée sur une préparation membranaire de rein de boeuf selon une méthode adaptée de Crause P. *et al*., Molecular and Cellular Endocrinology, 1982, *28*, 529-541 et de Stassen . F.L. *et al., J.* Pharmacol. Exp. Ther., 1982, *233*, 50-54. Les composés selon l'invention inhibent la fixation de l'arginine vasopressine tritiée aux récepteurs V₂ des préparations membranaires. Les Cl₅₀ des composés selon l'invention sont faibles: elles varient de 5.10⁻⁷ à 10⁻⁹M.

L'activité agoniste ou antagoniste des récepteurs de la vasopressine des composés selon l'invention, administrés par voie orale, a été évaluée chez le rat normalement hydraté (souche Sprague-Dawley) selon la technique décrite dans Br. J. Pharmacol., 1992, 105, 787-791.

L'effet diurétique, observé en général pour les composés de formule (I)et, pour certains de ces composés, à des doses inférieures ou égales à 10 mg/kg, montre que les composés de formule (I) constituent une série de puissants antagonistes V₂.

Les composés selon l'invention sont actifs après administration par différentes voies, notamment par voie orale.

Aucun signe de toxicité n'est observé avec ces composés aux doses pharmacologiquement actives et leur toxicité est donc compatible avec leur utilisation médicale comme médicaments.

Les composés selon la présente invention permettent, soit de mimer, soit d'inhiber, de façon sélective, les effets de la vasopressine et/ou de l'ocytocine. Parmi ces composés les antagonistes des récepteurs de la vasopressine peuvent intervenir sur la régutation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également étre utilisés dans le traitement de l'énurésie, et dans la régulation de l'hémostase: traitement de l'hémophilie, du syndrome de Von Willebrand, antidote des agrégants plaquettaires, Laszlo F.A., Pharmacol. Rev., 1991, *43*, 73-108. Drug Investigation, 1990, 2 (suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité (Vasopressin: Gross P. *et al*. ed. John Libbey Eurotext, 1993, en particulier 243-257 et 549-562. Laszlo F.A. and Laszlo F.A. Jr., Clinical perspectives for vasopressin antagonists, Drug News Perspect., 1993, 6 (8) ; North W.G., J. Clin. Endocrinol., 1991, *73*, 1316-1320. Legros J.J. *et al.,* Prog. Neuro-Pharmacol. Biol: Psychiat., 1988, *12*, 571-586 ; Andersson K.E. *et al*., Drugs Today, 1988, *24 (T),* 509-528 ; Stump D.L. et al., Drugs, 1990, 39,38-53 ; Caltabiano S. et al., Drugs Future, .1988, *13*, 25-30 ; Mura Y. *et al.,* Clin. Nephrol. 1993, *40,* 60-61; Faseb J., 1994, *8* (5), A587 : 3398).

Les molécules antagonistes V₂ à profil aquarétique possèbent un large éventail d'indications thérapeutiques et constituent une innovation majeure dans les traitements de l'insuffi cardiaque, des hyponatrémies, des désordres hydriques, des rétentions d'eau, etc. Ce type de composé peut remplacer avantageusement les diurétiques classiques dans toutes les pathologies où ils sont préconisés chez l'homme et chez l'animal. On peut aussi envisager avec de telles molécules le traitement de l'hypertension en association avec des antihypertenseurs d'autres classes thérapeutiques comme par exemple des β-bloquants, des inhibiteurs de l'enzyme de conversion ou encore des antagonistes des récepteurs de l'angiotensine II.

Ainsi les composés selon l'invention sont utiles notamment dans le traitement des affections des systèmes nerveux central et périphérique, du système cardiovasculaire, du système endocrinien et hépatique, de la sphère rénale, de la sphére gastrique, intestinale et pulmonaire, en ophtalmologie et dans les troubles du comportement sexuel, chez l'homme et chez l'animal.

La présente invention a donc également pour objet des compositions pharmaceutiques contenant une dose efficace d'un composé selon l'invention ou d'un sel, solvate ou hydrate pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, infra-veineuse, topique, intratrachéale, intranasale, transdermique, rectale ou intraocculaire, les principes actifs de formule (I) ci-dessus, ou leurs sels, solvates ou hydrates éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale,intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils' aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à dès suppositoires qui sont préparés avec des liants fondant à la température rectale, par exemple du beurre de cacao ou des polyéthyléneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propyléneglycol ou le butylèneglycol.

Le principe actif peut être formulé également sons forme de microcapsules, éventuellement avec un ou plusieurs supports ou additifs, ou bien avec des matrices telles qu'un polymère ou une cyclodextrine (patch, formes à libération prolongée).

Les compositions selon l'invention peuvent être utilisés dans, le traitement ou la prévention de différentes affections vasopressine-dépendantes ou ocytocine-dépendantes ainsi, que dans les dysfonctionnements de la sécrétion de la vasopressine ou d'ocytocine, les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome, de Von Willebrand ; les affections du systéme nerveux central, la migraine, le vasospasme cérébral, l'hémorragie cérébrale, les oedèmes cérébraux, la dépression, l'anxiété, la boulimie, les états psychotiques, les troubles de la mémoire par exemple les rénopathies et les dysfonctionnements rénaux comme les oedèmes, le vasospasme rénal, la nécrose du cortex rénal, le syndrome néphrotique, les hyponatriémies, l'hypokaliémie, le diabète, le syndrome de Schwartz-Bartter ou la lithiase rénale ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabéte insipide et l'énurésie; les affections du système hépatique tel que les cirrhoses du foie ; les ascites abdominales et tous les désordres provoquant une d'eau anormale; les désordres surrénaliens (maladie de Cushing) et en particulier l'hypercorticisme et l'hyperaldosteronémie, les divers désordres pancréatiques et la régulation du métabolisme lipidique en particulier avec les antagonistes de l'ocytocine. Les compositions selon l'invention peuvent également être utilisés dans, le traitement des troubles du comportement sexuel, dans la surcharge pondérale ou l'excès de poids et l'obésité en remplaçant avantageusement les diurétiques classiques déjà utilisés pour cette indication. Chez la femme, les compositions selon l'invention peuvent étre utilisées pour traiter la dysménorrhée ou le travail prématuré. On peut également utiliser les compositions selon l'invention dans le traitement des cancers pulmonaires à petites cellules, des encéphalopathies hyponatriémiques, de la maladie de Raynaud, du syndrome de Menière, du syndrome pulmonaire, du glaucome et de la prévention de la cataracte et dans les traitements postopératoires, notamment après une chirurgie abdominale, cardiaque ou hémorragique.

Les compositions de la présente invention peuvent contenir, à côté des produits de formule ni ci-dessus ou de leurs sels, solvates, ou hydrates pharmaceutiquement acceptables, d'autres principes actifs qui peuvent étre utiles dans le traitement des troubles ou maladies indiquées ci-dessus.

Ainsi; la présenté, invention a également : pour objet des compositions pharmaceutiques contenant plusieurs principes actifs en association dont l'un est un composé selon l'invention.

Ainsi, selon la présente invention,' on peut préparer des compositions pharmaceutiques contenant un composé selon l'invention associé à un composé agissant sur le système rénine-angiotensine tel qu'un inhibiteur de l'enzyme de conversion, un antagoniste de l'angiotensine II, un inhibiteur de la rénine. On peut également associer composé selon l'invention, par exemple, avec un vasodilatateur périphérique, un inhibiteur calcique, un béta-bloquant, un alpha-1-bloquant ou diurétique. De telles compositions seront utiles en particulier dans le traitement de l'hypertension ou de la défaillance cardiaque. On peut également associer deux composés selon l'invention: un antagoniste spécifique du récepteur V₁ à un antagoniste spécifique de l'ocytocine ou un antagoniste V₁ et un antagoniste V₂ ou un antagoniste V₂ et un agoniste V₁.

De façon avantageuse les compositions de la présente invention contient un produit de formule (IA) ci-dessus ou un ses sels, solvates ou hydrates pharmaceutiquement acceptable. Chacun de ces composés peut également être associé à un antagoniste spécifique de l'angiotensine II de préférence à l'irbésartan.

Ces associations permettront de renforcer les activités thérapeutiques des composés selon l'invention.

Les PREPARATIONS et EXEMPLES suivants illustrent l'invention sans toutefois la limiter.

Les spectres de résonance magnétique nucléaire ont été effectués dans le DMSO-d6 sauf mention contraire, à 200 MHz et les déplacements chimiques sont exprimés en p.p.m.

Les abréviations utilisées ci-aprés sont les suivantes:
s = singulet
m = multiplet
t = triplet
q = quadruplet
d = doublet

### PREPARATION 1 Alcools de formule (IIIB)

### 5-Ethoxy-3-spiro-(4-hydroxycyclohexane)indolin-2-one. Composé (IIIB1)

On chauffe à 40°C pendant 3 heures une solution de 22 g de 5-éthoxy-3-spiro-(4-méthoxyméthyloxycyclohexane)indolin-2-one préparée selon EP 636608 dans 130 ml de méthanot et 9 ml d'acide chlorhydrique concentré (36 %). On refroidit le mélange réactionnel, puis successivement on essore, rince à l'éther diéthylique et sèche le précipité pour obtenir l'isomère polaire du produit attendu; F = 225°C. On ajoute 50 ml d'eau au filtrat, puis successivement on évapore le méthanol, extrait au dichlorométhane, lave les phases organiques à l'eau, sèche et évapore pour obtenir le produit attendu sous forme d'un mélange d'isomères; F = 170°C.

### 5-Chloro-3-spiro-(4-hydroxycyclohexane)indolin-2-one. Composé (IIIB2)

On procède selon le même mode opératoire que précédemment à partir de la 5-chloro-3-spiro-(4-méthoxyméthyloxycyclohexane)indolin-2-one préparée à partir de 5-chloroindolin-2-one selon la méthode décrite dans EP 636608. On isole après extraction au dichlorométhane le produit attendu sous forme d'un mélange d'isomères; F = 260°C.

### PREPARATION 2 Hydrazides de formule (VI)

### N'-(4-Ethoxyphényl)-4,4-éthylènedioxycyclohexanecarbohydrazide. Composé (VI.I)

On ajoute à -40°C, 1,65 ml de chloroformiate d'isobutyle à un mélange de 2,63 g de 4,4-éthylénedioxycydohexanoate de sodium dans 20 ml de tétrahydrofurane puis 1,8 ml de triéthylamine. On ague le mélange réactionnel pendant 2 heures à 0°C, puis on ajoute à -20°C, 2,4 g de chlorhydrate de 4-éthoxyphénylhydrazine, on agite le mélange réactionnel pendant 2 heures à 0°C puis on ajoute 100 ml d'eau et on extrait à l'acétate d'éthyle. Les phases organiques sont lavées successivement à l'eau, avec une solution d'hydrogénosulfate de potassium (pH=2), avec une solution saturée de carbonate de potassium, séchées sur sulfate de magnésium et évaporées. On obtient le produit attendu après cristallisation dans l'éther diéthylique ; F = 158°C.

### N'-Phényl-4,4-éthylènedioxycyclohexanecarbohydrazide. Composé (VI.2)

De la même manière, on isole le composé (VI.2) à partir de la phénylhydrazine ; F =158°C.

### PREPARATION 3 Acétals de formule (VB)

### 5-Ethoxy-3-spiro-(4,4-éthylènedioxycyclohexane)indolin-2-one. Composé (VB1)

A -50°C, on ajoute 2,15 ml d'une solution de butyllithium 1,6M dans l'hexane à une suspension de 1 g de l'hydrazide (VI.1) dans 16 ml de tétrahydrofurane. On agite le mélange réactionnel pendant 15 minutes et on ajoute 16 ml de tétraline. On distille le tétrahydrofurane et on chauffe à 180°C pendant 45 minutes. On ajoute alors à température ambiante 20 ml d'acétate d'éthyle, puis successivement on lave à l'eau, sèche la phase organique sur sulfate de magnésium, distille les solvants sous vide et on chromatographie le résidu sur gel de silice en éluant avec un mélange cyclohexane/acétate d'éthyle 7/3 (v/v). On isole le produit attendu par cristallisation dans l'éther diéthylique ; F = 183°C

Le même produit est également obtenu par réaction de 5-éthoxy-3-spiro-(4-oxocyclohexane)indolin-2-one (composé (IIB1)) avec l'éthylèneglycol dans le cyclohexane en présence de tamis moléculaire 5 Å et d'acide paratoluènesulfonique en quantité catalytique.

### 5-Ethoxy-3-spiro-(4,4-propylènedioxycyclohexane)indolin-2-one. Composé (VB2)

On procède selon le même mode opératoire décrit précédemment pour la préparation du composé (VB1) à partir de l'hydrazide correspondant ou par réaction de 5-éthoxy-3-spiro-(4-oxocyclohexane)indolin-2-one (composé (IIB1)) avec du 1,3-propane-diol dans le cyclohexane en présence de tamis moléculaire 5 Å et d'acide paratoluénesulfonique en quantité catalytique ; F = 216°C.

### 3-Spiro-(4,4-éthylènedioxycyclohexane)indolin-2-one. Composé (VB3)

on, procède selon le mode opératoire décrit précédemment pour la préparation du composé (VI) à partir de l'hydrazide (VI.1) correspondant ; F = 218°C.

### PREPARATION 4 Cétones de formule (IIB)

### 5-Ethoxy-3-spiro-(4-oxocyclohexane)indolin-2-one. Composé (IIB1)

On dissout 3,8 g de 5-éthoxy-3-spiro-(4-hydroxycyclohexane)indolin-2-one (IIIB1) (mélange d'isomères) et 5,8 ml de pyridine dans 250 ml d'acétate d'éthyle et on ajoute 6,3g de chlorochromate de pyridinium adsorbé sur 29 g d'alumine neutre. On agite ensuite le mélange réactionnel à 25°C durant 16 heures, puis on filtre et on évapore le solvant du filtrat On isole 3,4 g du, produit attendu après recristallisation en présence de charbon actif dans le toluène ; F = 168°C. Le même produit est préparé par hydrolyse chlorhydrique du composé VB1.

### 5-Chloro-3-spiro-(4-oxocyclohexane)indolin-2-one. Composé (IIB2)

On prépare ce composé selon le même mode opératoire que pour la préparation du composé (IIB1) à partir de 5-chloro-3-spiro-(4-hydroxycyclohexane)indolin-2-one (IIIB2); F=220°C

### PREPARATION 5 Réactifs de formule (2)

### Chlorure de 2-méthoxy-4-N-tert-amylcarbamoylbenzènesulfonyle. Réactif (2).1

a) N-*tert*-amyl(3-méthoxy-4-nitro)benzamide
   On ajoute à 10°C, 30 ml de *tert*-amylamine à une solution de 27 g de chlorure de 3-méthoxy-4-nitrobenzoyle (obtenu à partir de 25 g d'acide correspondant et de chlorure de thionyle à reflux pendant 4 heures suivi d'une évaporation sous vide) dans 250 ml de dichlorométhane. On agite le mélange réactionnel durant 30 minutes à 20°C, puis on ajoute 100 ml d'une solution d'acide chlorhydrique 1 N, décante, lave et séche la phase organique sur sulfate de magnésium, puis, on évapore le solvant et on chromatographie le résidu sur gel de silice en éluant au dichlorométhane pour obtenir 31 g du produit attendu; F = 85°C. De la même manière et à punir de N-*tert* butylamine, on prépare le N-tert-butyl(3-méthoxy-4-nitro)benzamide ; F = 118°C.
b) N-*tert*-amyl-(3-méthoxy-4-amino)benzamide
   On chauffe à reflux pendant 3 heures un mélange de 31 g de N-*tert*-amyl-(3-méthoxy-4-nitro)benzamide obtenu en a), 20 g de palladium sur charbon à 10%, 76 ml de cyclohexène dans 310 ml d'éthanol. On filtre, évapore le filtrat pour obtenir 25 g du produit attendu; F = 108°C.
   De la méme manière, à partir du composé N-*tert*-butyl-(3-méthoxy-4-nitro) benzamide on prépare le N-*tert*-butyl-(3-méthoxy-4-amino)benzamide; F = 160°C.
c) Chlorure de 2-méthoxy-4-*tert*-amylcarbamoylbenzénesulfonyle
   On ajoute à 0°C une solutionde 7,9 g de nitrite de sodium dans 31 ml d'eau à une solution de 25 g de N-*tert*-amyl-(3-méthoxy-4-amino)benzamide dans 103 ml d'acide acétique et 187 ml. d'acide chlorhydrique à 36 %. On agite le mélange réactionnel pendant 1 heure à 0°C puis on ajoute cette solution conservée à 0°C à une suspension de 6,8 g de chlorure cuivrique, dans 25 ml d'eau et 140 ml d'acide acétique saturée à 0° Cpar environ 69 g de dioxyde de soufre. On agite le mélange réactionnel à 0°C pendant 3 heures puis à 20°C pendant 16 heures et on coule le milieu sur 750 g de glace en agitant ensuite pendant 1 heure à 20°C. On essore puis successivement on rince le précipité à l'eau, sèche sous vide durant 48 heures pour obtenir 19 g du produit attendu; F = 104°C.

### Chlorure de 4-N-tert-butylcarbamoyl-2-méthoxybenzènesulfonyle. Réactif (2).2

De la même manière, à partir de N-*tert*-butyl(3-méthoxy-4-amino)benzamide, on isole le réactif attendu ; F = 148°C.

### Chlorure de 2-méthoxy-4-benzyloxycarbonylbenzènesulfonyle. Réactif (2).3

En utilisant la même réaction que précédemment et, à partir de l'ester benzylique de l'acide 4-amino-3-méthoxybenzoïque (F = 72°C issu de la réduction du dérivé nitré correspondant par l'étain en milieu chlorhydrique; F = 88°C), on isole le réactif attendu; F = 55°C.

### N-tert-butyl-4-bromométhyl-3-méthoxybenzamide. Réactif (2).4

On agite à 30°C sous irradiation du spectre visible pendant 48 heures un mélange de 3 g de N-*tert*-butyl-4-méthyl-3-méthoxybenzamide, g de N-bromosuccinimide, 0,16 g de péroxyde de benzoyle dans 40 ml de tétrachlorure de carbone. On évapore le solvant, puis succéssivement on ajoute 25 ml d'eau, extrait à l'éther diéthylique, sèche sur sulfate de magnésium, évapore le solvant et chromatographie le résidu sur gel de silice en éluant avec un mélange cyclohexane/acétate d'éthyle 8/2 (v/v). On isole le réactif attendu après cristallisation dans l'éther isopropylique; F = 114°C.

### PREPARATION 6. Alcools protégés de formule (IVA)

### 5-Ethoxy-3-spiro-(4-méthoxyméthyloxycyclohexane)-1-[(4-N-tert-butylcarbamoyl-2-méthoxybenzènesulfonyl]indolin-2-one. Composé (IVAI)

On ajoute 0,283 g de *tert*-butylate de potassium à une solution refroidie à -40°C de 5-éthoxy-3-spiro-(4-méthoxyméthyloxycyclohexane)indolin-2-one, (composé de formule (IVB)) préparé selon EP 636608, dans 80 ml de tétrahydrofurane. On laisse remonter la température à 0°C puis refroidit le mélange à - 40°C et ajoute 0,73 g de chlorure de (2-méthoxy-4-N-*tert*-butylcarbamoy) benzènesulfonyle dans 7 ml de tétrahydrofurane. On agite le mélange réactionnel pendant 2 heures à température ambiante, puis successivement on ajoute 20 ml d'eau, extrait à l'acétate d'éthyle, séche sur sulfate de magnésium, évapore le solvant et purifie l'huile obtenue par chromatographie sur gel de silice en éluant avec un mélange cyclohexane/acétate d'éthyle 8/2 (v/v). On isole l'isomère le moins polaire du produit attendu; F = 165°C puis l'isomère polaire ; F = 156°C.

### PREPARATION 7 Alcools de formule (IIIA)

### 5-Ethoxy-3-spiro-(4-hydroxycyclohexane)-1-[(4-N-tert-butylcarbamoyl-2-méthoxy benzènesulfonyl]indolin-2-one. Composé (III A1)

On chauffe à 50°C pendant 1 heure un mélange de l'isomère polaire du composé (IVA1) dans 1,2 ml de méthanol et 0,24 ml d'acide chlorhydrique concentré (36 %). On ajoute 8 ml d'eau au mélange réactionnel, puis successivement on extrait au dichlorométhane, sèche les phases organiques sur sulfate de magnésium et on évapore les solvants. On obtient le produit attendu après purification par chromatographie sur gel de silice en éluant au dichlorométhane ; F = 268°C. (isomère polaire)

De la même manière, à partir de l'isomère le moins polaire préparé selon (IVA1), on isole l'isomère le moins polaire du produit attendu; F = 130°C (hémihydrate). Composé (IIIA2)

### PREPARATION 8 Cétones de formule (IIA)

### 5-Ethoxy-3-spiro-(4-oxocyclohexane)-1-[4-(4-N-tert-butylcarbamoyl)-2-methoxybenzènesulfonyl]indolin-2-one. Composé (IIA1)

On ajoute à -40°C, 0,38 g de *tert*-butylate de potassium à une solution de 0,8 g de 5-éthoxy-3-spiro-(4-oxocyclohexane)indolin-2-one (composé (IIB1)) dans 15 ml de trétrahydrofurane et on agite le mélange réactionnel pendant 15 minutes à 0°C. A -40°C, on ajoute 0,98 g de chlorure de 2-méthoxy-4-(4-N-*tert*-butylcarbamoyl) benzènesulfonyle dissout dans 10 ml de trétrahydrofurane et agite le mélange réactionnel à 20°C pendant 8 heures. On ajoute 30 ml d'eau, évapore le solvant sous pression réduite, extrait au dichlorométhane, sèche sur sulfate de magnésium et évapore le solvant sous pression réduite. On isole le produit attendu après une purification par chromatographie sur une colonne de gel de silice en éluant avec un mélange de cyclohexane/acétate d'éthyle 8/2 (v/v) et une recristallisation dans un mélange de cyclohexane/acétate d'éthyle 3/7 (v/v) ; F = 120°C. Le même composé est également obtenu par oxydation du composé (IIIA1) dans les conditions décrites dans la PREPARATION 4.

De la même manière, à partir des chlorures de sulfonyle et des indolin-2-one correspondants, on isole : **5-Ethoxy-3-spiro-(4-oxocyclohexane)-1-[4-(4-N-*tert*-amylcarbamoyl)-2-méthoxybenzènesulfonyl]indolin-2-one.**
Composé (IIA2) ; F = 191 °C.

### 5-Chloro-3-spiro-(4-oxocyclohexane)-1-[4-(4-N-tert-butylcarbamoyl)-2-méthoxybenzènesulfonyl]indolin-2-one hémihydrate. Composé (IIA3) ; F = 262°C.

### PREPARATION 9 Indolin-2-one de formule (II'B)

### 5-Ethoxy-3-spiro-[4-(2-formyléthylidène)cyclohexane])indolin-2-one. Composé (II'BI)

(II'B1) : R₁=OC₂H₅; R₂ = H ; R₃ = 2-OCH₃ ;

Y'₁ + Y'₂ == CHCH₂CHO

A une solution de 6 g de bromure de (3,3-diisopropyloxy)-propyl triphénylphosphonium (préparé dans Synthesis, 1988, 395) dans 100 ml de tétrahydrofurane, on ajoute à 0°C, 24 ml d'une solution 1M de bistriméthylsilylamidure de sodium dans le tétrahydrofurane. On agite le mélange réactionnel pendant 1 heure et demie puis on additionne 2,1 g du composé (IIB2) dans 20ml de tétrahydrofurane à -60°C et agite pendant 12 heures à 20°C. On évapore le solvant, isole le diisopropylacétal du produit attendu par chromatographie sur une colonne de gel de silice en éluant avec un mélange d'acétate d'éthyle/cyclohexane 25/75 (vlv) et (hydrolyse pendant 2 heures à 20°C dans un mélange de 3,5 ml de diméthylcétone, 3,5 ml d'eau et 0,02 ml d'acide chlorhydrique concentré. On extrait à l'acétate d'éthyle, lave la phase organique avec une solution saturée de bicarbonate de sodium, sèche sur sulfate de sodium et isote le produit attendu après évaporation du solvant.
RMN¹H= 10,1 (s,1H) ; 9,6 (s,1H) ; 6;95 (s,1H); 6,7 (s, 2H) ; 5,35 (t,1H); 4 (q, 2H) ; 3,2 (d, 2H) ; 2,6 (m, 2H) ;2,3 (m, 2H) ; 1,65 (m, 4H) ; 1,25 (t, 3H);

### 5-Ethoxy-3-spiro-(4-cyanocyclohexane)indolin-2-one Composé (II'B2)

A une suspension de 0,25 g de 5-éthoxy-3-spiro-(4-oxocydohexane)indolin-2-one dans 5 ml de 1,2-diméthyloxyéthane et 1 ml d'éthanol, on ajoute à -10°C 0,2 g de tosylméthylisonitrile et 0,32 g de *tert*-butylate de potassium. On agite à 15°C pendant 3 heures, ajoute 5 ml d'une solution saturée de chlorate d'ammonium, extrait à l'acétate d'éthyle, sèche sur sulfate de sodium et évapore le solvant sous pression réduite. On isole le produit attendu (mélange d'isomères) après une chromatographie sur une colonne de gel de silice en éluant avec un mélange de dichlorométhane/méthanol 98/2 (v/v) et une recristallisation dans le toluène ; F=174°C.

### 5-Chloro-3-spiro-(4-cyanocyclohexane)indolin-2-one. Composé (II'B3)

A une solution de 1 g du Composé (IIB2) dans 20 ml de diméthylsufoxyde, on ajoute à 16°C, 0,86 g de tosylméthylisonitrile puis 1,35 g de *tert*-butylate potassium. On agite à 20°C pendant 2 heures puis on ajoute 40 ml d'une solution aqueuse à 5 % de chlorure d'ammonium, extrait au dichlorométhane, sèche sur sulfate de sodium et évapore le solvant sous pression réduite. On chromatographie sur une colonne de gel de silice en éluant au dichlorométhane, reprend à l'éther diéthylique, et séche à 50°C sous pression réduite On isole ainsi le produit attendu (mélange d'isomères) ; F = 186°C.

### 5-chloro-3-spiro-[4-(2-aminoéthyl)cyclohexane]indolin-2-one Composé (II'B4)

**a) 5-Chloro-3-spiro-(4-cyanométhylidènecyclohexane)indolin-2-one isomére A**
   **5-chloro-3-spiro-(4-cyanométhylcyclohex-3-ène)indolin-2-one isomère B**
   d'après Synthesis, 1977,629
   A 10°C, sous atmosphére inerte, on ajoute à 0,7 g du Composé (IIB2) dans 10,5 ml d'acétonitrile, 0,4 g de potasse en poudre. On chauffe lentement jusqu'à une température de 80°C puis on refroidit avant d'ajouter 20 ml d'acide chlorhydrique 0,5 N puis 80 ml d'acétate d'éthyle. On sèche la phase organique sur sulfate de sodium, évapore à sec, chromatographie sur une colonne de gel de silice en éluant avec un mélange cyclohexane/acétate d'éthyle 90/10 (v/v). On isole deux isomères:
   - Composé A : le moins polaire; F = 78°C
   - Composé B : le plus polaire ; F = 155°C
**b) 5-Chloro-3-spiro-[4-(2-aminoéthyl)cyclohexane]indolin-2-one**
   On hydrogéne sous une pression de 2 MPa d'hydrogéne, pendant 24 heures à 32°C, 0,14 g du Composé A et 0,11 g du Composé B dans 50 ml d'une solution d'ammoniac à 10 % dans le méthanol en présence de 0,30 g de nickel de Raney humide. On refroidit à 10°C, filtre le catalyseur, évapore les solvants pour obtenir le produit attendu sous forme d'un mélange de deux isomères; F = 90°C:

### PREPARATION 10 Composes de formule (II'A)

### 5-Chloro-1-[4-N-tert-butylcarbamoyl-2-méthoxybenzénesulfonyl]-3-spiro-[4-(méthoxyméthylidéne)cyclohexane]indolin-2-one. Composé (II'A1)

On opère selon la méthode décrite dans J. Am. Chem. Soc., 1967, *89*, 1492.
A solution de diisopropylamidure de lithium (préparée à 4°C par addition de 0,6 ml d'une solution de 1,6M de butyllithium dans l'hexane à 0,13 ml de diisopropylamine dans 4 ml d'éther diéthylique), on ajoute à 0°C 0,66 g de chlorure de (méthoxy méthyl)triphénylphosphonium et agite le mélange réactionnel pendant 30 minutes. On refroidit à -30°C, additionne 0,250 g du composé (IIA3) dans 4 ml de tétrahydrofurane, agite pendant 8 heures à 10°C, hydrolyse et extrait à l'acétate déthyle. On lave la phase organique avec une solution saturée de chlorure de sodium, sèche sur sulfate de magnésium, évapore les solvants sous pression réduite et isole le produit attendu par chromatographie sur une colonne de gel de silice en éluant avec une mélange cyclohexane/acétate d'éthyle 85/15 (v/v);
F = 180°C.

### 5-Chloro-1-[4-N-tert-butylcarbamoyl)-2-méthoxybenzènesulfonyl]-3-spiro-[4-formylcyclohexane]indolin-2-one Composé (II'A2)

On agite à 20°C pendant une heure un mélange de 0,12 g du composé (II'A1) dans 4 ml de tétrahydrofurane et 1 ml d'une solution aqueuse à 30% d'acide perchlorique. On ajoute 20 g de glace, extrait à l'acétate d'éthyle, sèche sur sulfate de sodium et isole le produit attendu après évaporation des solvants sous pression réduite ; F =198°C.

### PREPARATION 11 Composés de formule (II"B)

### 5-Ethoxy-3-spiro-[4-(2-morpholinoéthylthio)cyclohex-3-éne]indolin-2-one Composé (II''B1)

a) Le 2-morpholinoéthanethiol est synthétisé selon le mode opératoire décrit dans JACS, 1948, 70, 950.
b) 5-Ethoxy-3-spiro-[4-(2-morpholinoéthylthio)cyclohex-3-ène]indolin-2-one
   A -10°C, on additionne à une solution de 0,5 g de 5-éthoxy-3-spiro-(4-oxocyclohexane) indolin-2-one (Composé IIB1) dans 20 ml de dichlorométhane 1,13 g de 2-morpholinoéthanethiol puis 0,6 ml de trifluoroborane éthérate. On chauffe 5 heures à reflux. A 10°C, on ajoute 10 ml d'une solution aqueuse à 5 % en K₂CO₃ puis extrait au dichlorométhane. On décante, séche sur Na₂SO₄, évapore le solvant Le résidu obtenu est chromatographié sur une colonne de gel de silice en éluant avec un mélange dichlorométhane/métanol 98/2 (v/v) pour fournir le composé attendu.
   RMN¹H = 6,7 (s, 2H) ; 6,55 (s, 1H) ; 5,7 (m, 1H); 3,9 (q, 2H) ; 3,55 (t, 4H); 2,85 (m, 2H) ; 2,5 (m, 2H) ; 2,35 (m, 6H) ; 2,1-1,8 (m, 2H); 1,55 (m, 1 H); 1,23 (t, 3H); 1,24 (m, 1H).

### PREPARATION 12 Indolin-2-ones de formule (I')

### 5-Ethoxy-3-spiro-[4-(3-morpholinopropylidène)cyclohexane]indolin-2-one Composé (I'1)

(I'1) : R₁ = OC₂H₅ ; R₂ = H

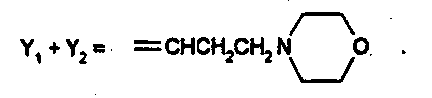

A une solution de 0,4 g du composé (II'B1) dans 10 ml de tétrahydrofurane, on ajoute 0,23 ml de morpholine, 0,42 9 de triacétoxyborohydrure de sodium, 0,075 ml d'acide acétique et agite le mélange réactionnel pendant 20 heures à 20°C. On ajoute 10 ml d'acide chlorhydrique 1N et extrait la phase aqueuse à l'éther diéthylique. La phase organique est écartée. La phase aqueuse est alcalinisée avec de l'hydroxyde de sodium 10N, extraite à l'acétate d'éthyle et séchée sur sulfate de sodium. On isole le composé attendu aprés évaporation du solvant sous pression réduite.
RMN¹H = 10,1 (s, 1H); 6,95 (s, 1H); 6,7 (s, 2H) ; 5,2 (t, 1H); 3,9 (q, 2H); 3,5 (m, 4H) ; 2,6-2,2 (m, 12H) ; 1,6 (m, 4H); 1,25 (t, 3H).

### 5-Ethoxy-3-spiro-[4-(3-morpholinopropyl)cyclohexane]indolin-2-one. Composé (T2)

(I'2):R₁=OC₂H₅ ; R₂=H;

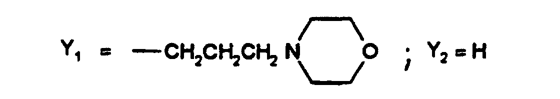

On hydrogène sous 1,5 MPa à 40°C pendant 24 heures 0,44 g du composé (I'1) dans 25 ml d'éthanol en présence, de 0,2 g de palladium sur charbon à 10%. On sépare le catalyseur par filtration, évapore le solvant et isole le produit attendu (mélange d'isomères) par chromatographie sur une colonne de gel de silice en éluant avec un mélange de dichlorométhane/méthanol 98,5/1,5 (v/v).
RMN¹H =10,1 (s, 0,3H) ; 9,95 (s, 0,7H) ; 7 (s, 0,3H) ; 6,84 (s, 0,7H) ; 6,75-6,65 (m, 2H) ; 3,9 (t, 2H); 3,5 (t, 4H); 2,3 (m, 6H); 1,8-1,15 (m, 16H);

### 5-Chloro-3-spiro-[4-(2-tert-butoxycarbonylaminoéthyl)cyclohexane] indolin-2-one. Composés (I'6) et (I'7)

(I'6) et (I'7): R₁ = 5-Cl ; R₂ = H ; Y₁ = -(CH₂)₂NHCOOC(CH₃)₃ ; Y₂ = H

A 0,25 g du Composé (II'B4) dans 5 ml de dioxane et 0,5 ml d'hydroxyde de sodium 2 N, on ajoute 0,26 g de di-*tert*-butyldicarbonate et 0,05 g d'oxyde de magnésium. On agite à 20°C pendant 16 heures puis on ajoute 20 ml d'eau et extrait avec de l'acétate d'éthyle. On sèche la phase organique sur sulfate de sodium, évapore les solvants, chromatographie sur une colonne de gel de silice en éluant avec du dichlorométhane. On isole le produit sous forme de 2 isomères.
- Composé (I'6) : le moins polaire; F = 85°C
- Composé (I'7): le plus polaire; F = 78°C

### 5-Chloro-3-spiro-[(4-tert-butoxycarbonylaminométhyl)cyclohexane]indolin-2-one. Composé (I'8)

(I'8): R₁ = 5-Cl; R₂= H; Y₁ = CH₂NHCOOC(CH₃)₃ ; Y₂ = H

On hydrogène sous 2,5 MPa de pression d'hydrogène pendant 48 heures à 28°C, 0,21 g du composé (II'B3) dans 20 ml d'une solution d'ammoniac à 14 % dans le méthanol en présence de 0,5 g de Nickel de Raney humide. A 15° C, on élimine le catalyseur par filtration, évapore le filtrat, reprend le résidu avec 5 ml de 1,4-dioxane, 0,5 ml d'eau, traite avec 0,34 ml d'hydroxyde de sodium 2N, 0,04 g d'oxyde de magnésium et 0,17 g de di-*tert*-butyldicarbonate. On agite à 20°C pendant 3 heures, ajoute 10 ml d'eau et extrait à l'acétate d'éthyle. On sèche la phase organique sur sulfate de sodium, évapore sous pression réduite et isole le produit attendu (mélange d'isomères) après chromatographie sur une colonne de gel de silice en éluant avec un mélange dichlorométhane/méthanol 99/1 (v/v);
F = 86°C

### PREPARATION 13 Réactifs aminés de formule (1)

### • 2-(4-Benzyloxypipéridino)éthylamine

1) N-(2=(4-Benzyloxypipéridino)éthyl)phtalimide
   On chauffe à 55°C pendant 8 heures un mélange de 4,5 g de N-(2-bromoéthyl)phtalimide, 3,2 g de 4-benzyloxypipéridine et 4,5 g de carbonate de potassium dans 40 ml d'acétonitrile. On filtre, extrait le filtrat à l'acétate d'éthyle, lave la phase organique avec trois fois 50 ml d'eau, sèche sur sulfate de magnésium et évapore le solvant sous pression réduite. Le composé attendu isolé sous forme d'huile est engagé tel quel dans l'étape suivante.
2) 2-(4-Benzyloxypipéridino)éthylamine
   On chauffe à reflux pendant 3 heures le composé précédent et 2,5 ml d'hydrate d'hydrazine dans 100 ml de méthanol. On refroidit le mélange réactionnel à 5°C, filtre et concentre le filtrat sous pression réduite. On ajoute 25 ml d'acide chlorhydrique 6N et chauffe à 50°C pendant 1 heure. On refroidit à 0°C, filtre et alkalinise le filtrat avec de l'hydroxyde de sodium concentré. On extrait au dichlorométhane, sèche sur sulfate de sodium anhydre et évapore le solvant sous pression réduite. On isole le produit attendu par distillation sous vide.
   Eb = 105-110°C sous 4 Pa.

### ● Acide 6-amino hexanoïque amide:

Préparé selon JACS, 1946, *68*, 1684 et Chem. Ber. 1959, *92*, 2616-2620.

### EXEMPLE 1

### 5-Ethoxy-1-[4-(N',N'-diéthyluréido)-2-méthoxybenzènesulfonyl]-3-spiro-(4-morphotinocyclohexane)indolin-2-one

(I):R₁=OC₂H₅; R₂ = H ; R₃=2-OCH₃; W = SO₂ ; R₄ = 4-NHCON(C₂H₅)₂ ; 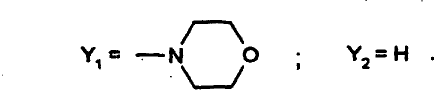 Y₂=H.

A 20°C, on ajoute à une solution de 0,25 g de 5-éthoxy-1-[4-(N',N'-diéthyluréido)-2-méthoxybenzènesulfonyl]-3-spiro-[4-oxocyclohexane)indolin-2-one (préparée dans EP 636608) dans 2,5 ml de 1,2-dichloroéthane et 0,04 g de morpholine, 0,150 g de triacétoxyborohydrure de sodium et 0,03 g d'acide acetique. On agite le mélange reactionnel pendant 16 heures à 20°C, puis on ajoute 4 ml d'une solution saturée de bicarbonate de sodium et on extrait à l'acétate d'éthyle. On sèche sur sulfate de magnésium, évapore le solvant sous pression réduite et on obtient un mélange d'isoméres du produit attendu qui est purifié par chromatographie sur une colonne de gel de silice en éluant avec du cyclohexane puis avec un mélange dichlorométhane/méthanol 98/2 (v/v). On isole l'isomère le moins polaire du produit attendu. (Chromatographie sur couche mince, silice, éluant dichlorométhane/ méthanol 93/7 (v/v), Rf = 0,55) ; F = 105°C.
puis l'isomère polaire (Rf = 0,46) ; F = 125°C.

De la même manière, en variant les cétones (IIA) et les amines, on obtient les composés des EXEMPLES 2 à 13 rassemblés dans le TABLEAU I suivant

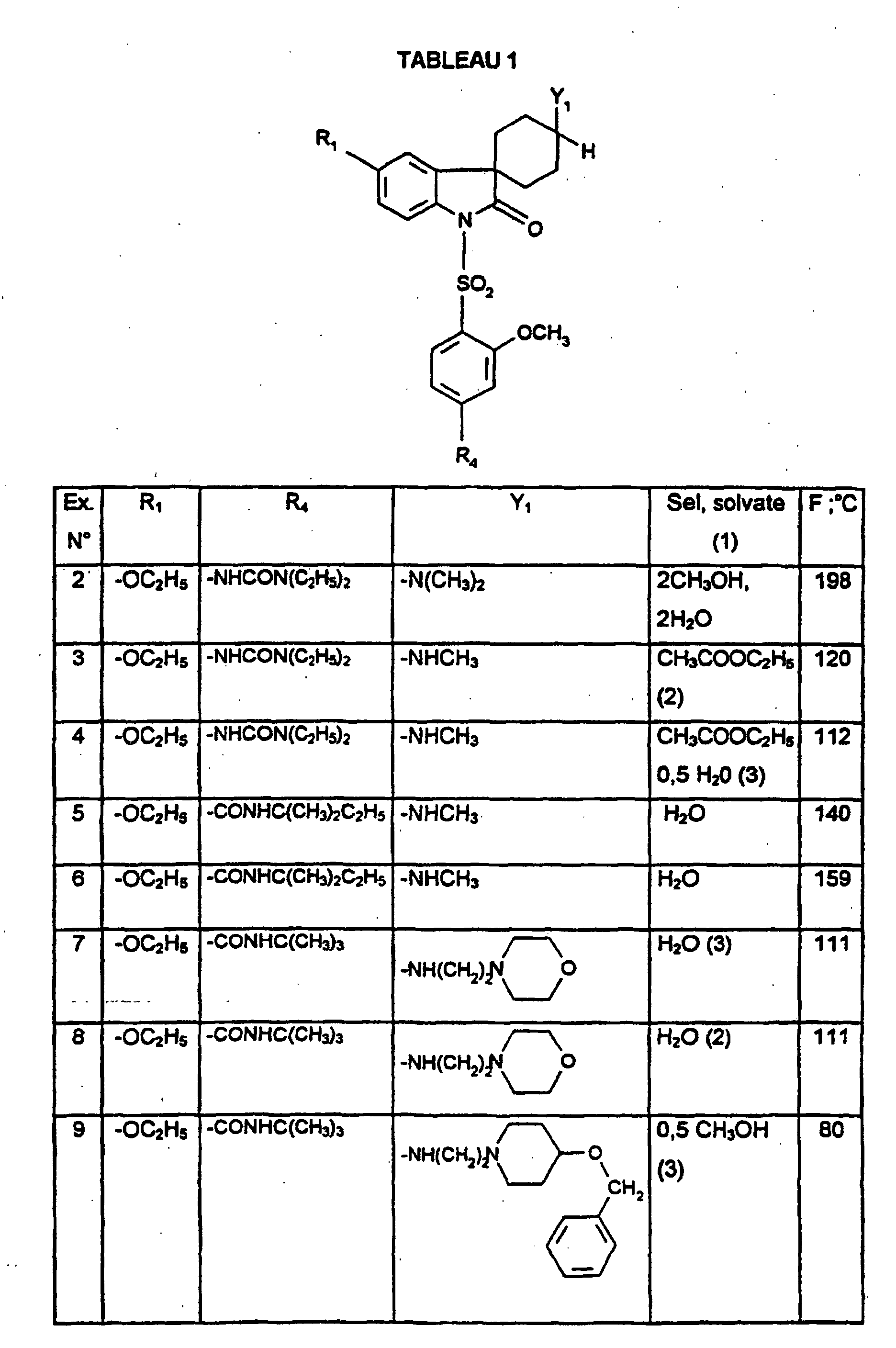

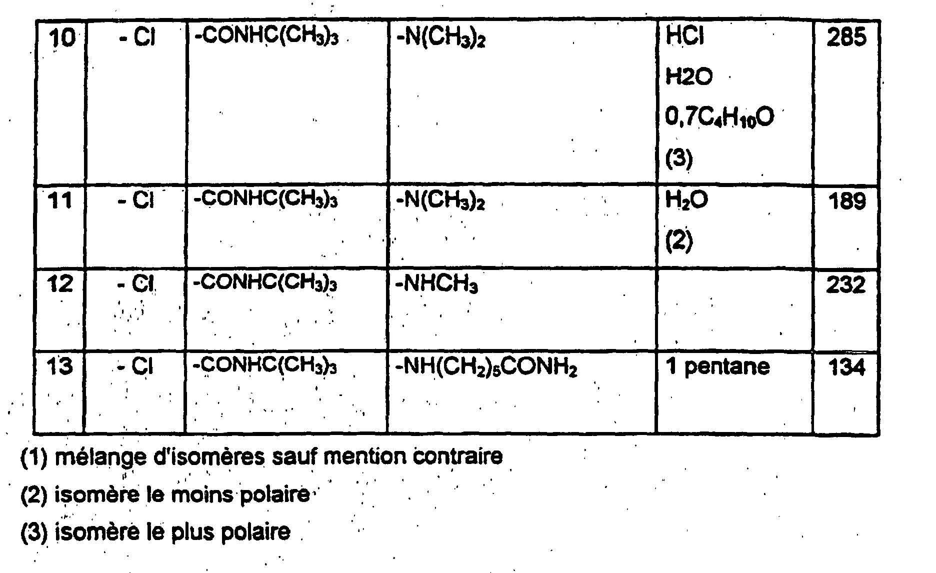

### EXEMPLE 14

### 5-Ethoxy-3-spiro-[4-(N-méthyl-2-(morpholinoéthyl)amino)cyclohexane]-1-[(4-N-tert-butylcarbamoyl-2-méthoxy)benzènesulfonyl]indolin-2-one.

(I): R₁ = OC₂H₅; R₂ = H; R₃=2-OCH₃; W=SO₂; R₄=4-CONHC(CH₃)₃; 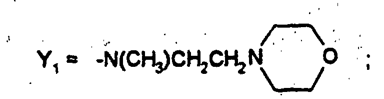 Y₂=H

A une solution refroidie à 5°C de 0,13 g du composé de l'EXEMPLE 7 dans 1,3 ml d'acétonitrile et 0,076 ml d'une solution aqueuse de formaldéhyde à 37%, on ajoute 0,04 g de çyanoborohydrure de sodium et 0,046 ml d'acide acétique. On agite le mélange réactionnel pendant 3 heures à 20°C et on ajoute 2 ml d'eau, 2 ml d'une solution saturée de bicarbonate de sodium et on extrait à l'acétate d'éthyle. On sèche sur sulfate de sodium anhydre et évapore le solvant sous pression réduite. On isole le dichlorhydrate pentahémihydraté du composé attendu après purification sur une colonne de gel de silice en éluant avec un mélange dichlorométhane/ méthanol 97/3 (v/v) et chlorhydratation dans l'éthanol; F = 222°C.

### EXEMPLE 15

### 5-Chloro-3-spiro-[4-(N-acétyl-5-carboxamido-pentylamino)cyclohexane]-1-[(4-N- tert-butylcarbamoyl-2-méthoxy)benzènesulfonyl]indolin-2-one.

(I): R₁=5-Cl; R₂=H; R₃=2-OCH₃; W=SO₂; R₄ =4-CONHC(CH₃)₃
Y₁ =-N(COCH₃)(CH₂)₅ CONH₂; Y₂=H.

A -30°C, on ajoute à 0,1 g de composé de l'EXEMPLE 13 en solution dans 2 ml de dichlorométhane et 0,05 ml de triéthylamine 0,014 g de chlorure d'acétyle. A 20°C, on ajoute 3 ml d'eau et extrait au dichlorométhane. On sèche la phase organique sur Na₂SO₄, évapore le solvant, recristallise le résidu dans un mélange cyclohexane/acétate d'éthyle 70/30 (v/v). On filtre, sèche à 20°C sous pression réduite pour obtenir le produit attendu; F=266°C.

### EXEMPLE 16

### 5-Ethoxy-1-[4-(N',N'-diéthyluréido)-2-(méthoxybenzènesulfonyl]-3-spiro-[(4-hydroxy-4-tert-butyloxycarbonylméthyl)cyclohexane]indolin-2-one.

(I): R₁=5-OC₂H₅; R₂=H; R₃=2-OCH₃; W=SO₂; R₄=4-NHCON(C₂H₅)₂;
Y₁=-CH₂COOC(CH₃)₃; Y₂=OH.

A -15°C, on ajoute 1,9 ml d'une solution 1,6 M de butyltithium dans l'hexane à 0,41 ml de diisopropylamine dans 3 ml de tétrahydrofurane. On refroidit vers -70°C et ajoute lentement 0,2 g d'acétate de tert-butyle puis 0,59 g de 5-éthoxy-1-[4-(N',N'-diéthyluréido)-2-méthoxybenzènesulfonyl]3-spiro-(4-oxo-cyclohexane) indolin-2-one (préparée dans EP 636608) dans le mélange de tétrahydrofurane. On agite à -60°C pendant la minutes et ajoute à cette température 5 ml d'une solution aqueuse saturée de chlorure d'ammonium. On extrait à l'acétate d'éthyle, sèche sur sulfate de magnésium et évapore le solvant sous pression réduite. Le composé attendu sous forme d'un mélange d'isomères est purifié par chromatographie sur une colonne de gel de silice en éluant successivement avec du dichlorométhane puis avec un mélange dichlorométhane/méthanol 99/1 (v/v). On isole l'isomère le moins polaire du composé attendu (Chromatographie sur couche mince, silice, éluant dichlorométhane/méthanol 95/5 (v/v), (Rf = 0,57) ; F = 135°C.
puis l'isomère polaire (Rf = 0,45); F = 140°C.

### EXEMPLE 17

### 5-Ethoxy-1-[4-(N',N'-diéthyluréido)-2-méthoxybenzènesulfonyl]-3-spiro-[(4-hydroxyl-carboxyméthyl)cyclohexane]indolin-2-one

(I) : W= SO₂ ; R₄ = 4-NHCON(C₂H₅)₂; Y₁ = -CH₂COOH ; Y₂ = OH.

On ajoute à -20°C, 0,5 ml d'acide trifluoroacétique à une solution de 0,05 g du composé de l'EXEMPLE 16, 0,2 ml-d'anisole dans 0,8 ml de dichlorométhane. On agite à 20°C pendant 5 heures et on évapore les solvants sous pression réduite. Le résidu est repris dans 3 ml d'eau et extrait à l'acétate d'éthyle. On sèche sur sulfate de magnésium et évaporé le solvant sous pression réduite.
A partir de l'isomère le moins polaire de l'EXEMPLE 16, on isole le composé attendu solvaté avec 1 mole d'acide trifluoroacétique ; F =145°C .
A partir de l'isomère le plus polaire du composé de l'EXEMPLE 16, on isole le composé attendu solvate avec 1 Mole de d'acide trifluoroacétique ; F = 142°C.

### EXEMPLE 18

### 5-Ethoxy-1-[(4-N-tert-butylcarbamoyl-2-méthoxy)benzènesulfonyl]-3-spiro-(4-tert-butylcarboxyméthylidènecyclohexane]indolin-2-one

(I) : R₁ = 5-OC₂H₅ ; R₂ = H ; R₃ = 2-OCH₃ ; ;W=SO₂; R₄ = 4-CONHC(CH₃)₃;
Y₁ + Y₂= =CH-COOC(CH₃)₃

A une solution refroidie à -70°C de diisopropyl amidure de lithium (préparée à 4°C par addition de 5,2 ml de butyllithium 1,6N dans l'hexane à 1,15 ml de diisopropyl amine dans 15ml de tétrahydrofurane) on additonne 1,25 ml de triméthylsilyl acétate de tert-butyle puis 2 g du composé (IIA1) dans 20 ml de tétrahydrofurane. On agite le mélange réactionnel pendant 30 minutes à 40°C puis on ajoute 20 ml d'une solution aqueuse d'acide chlorhydrique 3N. On extrait l'acétate d'éthyle, sèche sur sulfate de magnésium et évapore les solvants sous pression réduite. On isole le composé attendu après une chromatographie sur une colonne de gel de silice en éluant avec un mélange cyclohexane/acetate d'éthyle 80/20 (vlv) ;
F = 206°C.

### EXEMPLE 19

### 5-Chloro-1-[-4-N-tert-butylcarbamoyl)-2-méthoxy)benzènesulfonyl-3-spiro- (4-tert-butylcarboxyméthylidénecyclohexane)indolin-2-one

(I):R₁=5-Cl ; R₂=H; R₃ = 2-OCH₃ ; W = SO₂ ; R₄ = 4-CONHC(CH₃)₃
Y₁ + Y₂ = = CH-COOC(CH₃)₃

Préparé comme dans l'EXEMPLE 18 à partir du composé (IIA3) ; F =135°C.

### EXEMPLE 20

### 5-Ethoxy-1-[(4-N-tert-butylcarbamoyl-2-méthoxy)benzénesulfonyl]-3-spiro-(4-carboxyméthylidénecyclohexane)indolin-2-one

(I) : R₁ = 5-OC₂H₅ ; R₂ = H ; R₃ =2-OCH₃ ; W = SO₂; R₄ = 4-CONHC(CH₃)₃ ;
Y₁ + Y₂= =CHCOOH

A une solution refroidie à -30°C de 1,6 g du composé de l'EXEMPLE 18 dans 30 ml de dichiorométhane, on ajoute 14 ml d'acide trifluoroacétique et agite pendant 2 heures à 20°C. On évapore le mélange réactionnel sous pression réduite et on isole le composé attendu par cristallisation dans le pentane. (Hydrate) ; F = 190°C.

### EXEMPLE 21

### 5-Chloro-1-[(4-N-tert-butylcarbamoyl)-2-méthoxy)benzénesulfonyl]-3-spiro-(4-carboxyméthylidènecyclohexane)indolin-2-one

(I): R₁ = 5-Cl; R₂ = H ; R₃ = 2-OCH₃ ; W = SO₂ ; R₄ = 4-CONHC(CH₃)₃
Y₁ +Y₂ = = CHCOOH

Préparé comme dans l'EXEMPLE 20 à partir du composé de l'EXEMPLE 19;
F = 199°C.

### EXEMPLE 22

### 5-Ethoxy-1-[(4-N-tert-butylcarbamoyl-2-méthoxy)benzènesulfonyl]-3-spiro-(4-carboxyméthylcyclohexane)indolin-2-one

(I):R₁ =5-OC₂H₅ ; R₂=H ; R₃ = 2-OCH₃ ; W = SO₂; R₄=4-CONHC(CH₃)₃;
Y₁= -CH₂COOH; Y₂=H.

On hydrogène sous 1,5 MPa pendant 16 heures à 40°C, 0,8 g du composé de l'EXEMPLE 20 dans 20 ml d'acide acétique en présence de 0,1 g d'oxyde de platine. On sépare le catalyseur par filtration, évapore le filtrat sous pression réduite et isole le composé attendu par cristallisation dans le pentane. (Mélange d'isomères); F=192°C (H₂O).

### EXEMPLE 23

### 5-Ethoxy-1-[(4-N-tert-butylcarbamoyl-2-méthoxy)benzénesulfonyl]-3-spiro-(4-morpholinocarbonylméthylidènecyclohexane)indolin-2-one

(I) : R₁ = 5-OC₂H₅; R₂ = H; R₃ = 2-OCH₃; W = SO₂; R₄ = 4-CONHC(CH₃)₃;

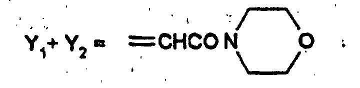

A une suspension de 0,6 g du composé de l'EXEMPLE 20 dans 4 ml d'acétonitrile, on ajoute à 5°C, 0,52 g d'hexafluorophosphate de benzotriazole-1-yl-oxy-*tris*-(diméthylamino)-phosphonium, 0,3 ml de triéthylamine puis 0,11 ml de morpholine.
On agite le mélange réactionnel pendant 3 heures à 20°C puis évapore le solvant sous pression réduite. On isole le composé attendu par chromatographie sur une colonne de gel de silice en éluant avec un mélange de dichlorométhane/méthanol 99/1 (v/v) et recristallisation dans l'acétate d'éthyle ; F = 238°C.

### EXEMPLE 24

### 5-Ethoxy-1-[(4-N-tert-butylcarbamoyl-2-méthoxy)benzènesulfonyl]-3-spiro-(4-(3-diméthylaminopropylaminocarbonylméthyl)cyclohexane)indolin-2-one

(I): R₁ = 5-OC₂H₅; R₂ = H ; R₃ = 2-OCH₃; W = SO₂; R₄=4-CONHC(CH₃)₃;
Y₁ = ―CH₂CONH(CH₂)₃N(CH₃)₂; Y₂=H

Préparé comme dans l'EXEMPLE 23 à partir du composé de l'exemple 22 et de 3-diméthylaminopropylamine. (Chlorhydrate hydrate) ; F = 71°C.

### EXEMPLE 25

### 5-Ethoxy-1-[(4-N-tert-butylcarbamoyl-2-méthoxy)benzènesulfonyl]-3-spiro-(4-morpholinocarbonylméthylcyclohexane)indolin-2-one

(I) : R₁ = 5-OC₂H₅ ;R₂=H;R₃ =2-OCH₃ ; W = SO₂ ; R₄ = 4-CONHC(CH₃)₃ ; 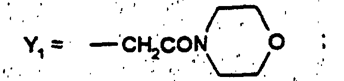 Y₂=H.

On hydrogène sous 1,5 MPa pendant 16 heures à 40°C 0,32 g du composé de l'EXEMPLE 23 dans 30 ml d'éthanol en présence de 0,3 g de palladium sur charbon à 10%. On le catalyseur par filtration, évapore le filtrat sous-pression rèduite et isole le composé attendu hémihydraté (mélange d'isomères) ; F =158°C.
Le même composé est obtenu, par réaction du composé de l'EXEMPLE 22 avec la morpholine dans les conditions décrites pour l'EXEMPLE 23.

### EXEMPLE 27

### 5-Chloro-1-[(4-N-tert-butylcarbamoyl-2-méthoxy)benzènesulfonyl]-3-spiro-[4-N-(4-N',N'-diméthylaminobutyl)carbamoylméthylidènecyclohexane]indolin-2-one.

(I): R₁ = 5-Cl; R₂=H; R₃=2-OCH₃; W=SO₂; R₄=4-CONHC(CH₃)₃
Y₁ + Y₂ = =CH-CONH(CH₂)₄N(CH₃)₂

Préparé comme dans l'EXEMPLE 23 à partir du composé de l'EXEMPLE 21 et de la 4-diméthylaminobutylamine; F= 170°C (H₂O).

### EXEMPLE 28

### 5-Chloro-1-[(4-N-tert-butylcarbamoyl-2-méthoxy)benzènesulfonyl]-3-spiro[4-N-(4-N',N'-diméthylaminobutyl)carbamoylméthylcyclohexane]indolin-2-one.

(I) : R₁ = 5-Cl ; R₂=H; R₃ = 2-OCH₃ ; W = SO₂ ; R₄ = 4-CONHC(CH₃)₃
Y₁=-CH₂-CONH(CH₂)₄ N(CH₃)₂ ; Y₂ = H

Préparé comme dans l'EXEMPLE 25 à partir du composé de l'EXEMPLE 27; F = 273°C (1HCl, 2C₂H₅OH).

### EXEMPLE 29

### 5-Ethoxy-1-[4-(N-tert -butylcarbamoyl)-2-méthoxybenzénesulfonyl)-3-spiro-[4-(3-morpholinopropyl)cyclohexane]indolin-2-one (Mélange d'isomères)

(I) : R₁ = 5-OC₂H₅; R₂ = H; R₃ = 2-OCH₃; W = SO₃; R₄ = 4-CONHC(CH₃)₃; 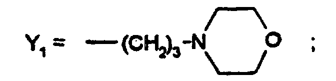 Y₂=H.

On ajoute 0,0374 g de *tert*-butylate de potassium à une solution refroidie à - 40°C de 0,113 g du composé (I'2) dissout dans 2 ml de tétrahydrofurane et laisse la température remonter à O°C. A -40°C on ajoute 0,097 g de chlorure de 4-(N-*tert*-butylcarbamoyl)-2-méthoxybenzènesulfonyle dissout dans 1 ml trétrahydrofurane. On agite le mélange réactionnel pendant 3 heures à -20°C puis successivement, on extrait à l'acétat d'éthyle, lave la phase organique par une solution saturée de chlorure d'ammonium, sèche sur sulfate de magnésium et évapore le solvant sous pression réduite. On isole la base du produit attendu par chromatographie sur une colonne de gel de silice en éluant avec un mélange de dichlorométhane/méthanol 99/1 (v/v) puis le chlorhydrate dihydraté par cristallisation dans l'éther diéthylique en présence d'acide chlorhydrique;
F = 184°C.

### EXEMPLE 31

### 5-Chloro-1-[4-(N-tert-butylcarbamoyl-2-méthoxy)benzénesulfonyl]-3-spiro-[4-(3-benzyloxypropylidène)cyclohexane]indolin-2-one

(I) : R₁ = 5-Cl; R₂ = H; R₃ = 2-OCH₃; W = SO₂; R₄ = 4-CONHC(CH₃)₃;

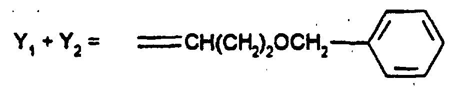

A -10°C, sous atmosphére inerte, on ajoute 3,8 ml dune solution de (bistriméthylsilyl)amidure de sodium 1 M dans le tétrahydrofurane à 1,9 g de bromure de (3-benzyloxypropyl)triphébnylphosphonium dans 30 ml de trétrahydrofurane. On agite à 20°C pendant 30 minutes puis additionne à - 70°C 1 g du composé (IIA3) en suspension dans 40 ml de trétrahydrofurane. On agite pendant 16 heures, ajoute à 5°C 20 ml d'une solution aqueuse de chlorure d'ammonium à 5 %, et extrait à l'acétate d'éthyle. On sèche la phase organique sur Na₂SO₄ , évapore les solvants, chromatographie le résidu sur une colonne de gel de silice en éluant avec un mélange cyclohexane/acétate d'éthyle 80/20 (v/v) et cristallise le produit attendu dans l'éther isopropylique; F = 138°C.

### EXEMPLE 33

### 1-[(4-N-tert-butylcarbamoyl-2-méthoxy)benzènesulfonyl]-3-spiro-[4-(3-hydroxypropyl)cyclohexane]indolin-2-one

(I): R₁=H; R₂= H ; R₃=2-OCH₃ ; W=SO₂ ; R₄=4-CONHC(CH₃)₃;
Y₁ = ―(CH₂)₃OH ; Y₂= H

On hydrogène sous 1,5 MPa à 25°C pendant 48 heures, 0,25 g du composé de l'EXEMPLE 31 dans 40 ml d'éthanol en présence de 0,2 g de palladium sur charbon à 10 %. On sépare le catalyseur par filtration, évapore le solvant et chromatographie le résidu sur une colonne de gel de silice en éluant avec un mélange cyclohexane/acétate d'éthyle 80/20 (v/v). On concrète le produit attendu dans le pentane; F = 133°C (0,5 H₂O).

### EXEMPLE 34

### 1-[(4-N-tert-butylcarbamoyl-2-méthoxy)benzènesulfony]-3-spiro-[4-(3-morpholinopropyl)cyclohexane]indolin-2-one

(I)R₁ = H ; R₂ = H; R₃ = 2-OCH₃ ; W = SO₂ ; R₄ = 4-CONHC(CH₃)₃ ; 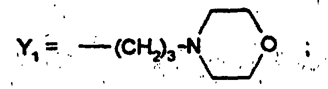 Y₂=H.
**a)1[(4-N-*tert*-butylcarbamoyl-2-méthoxy)benzènesulfonyl]-3-spiro-[4-(3-tosyloxypropyl)cyclohexane]indolin-2-one**
   A -10°C, on ajoute 0,03 g de chlorure de tosyle à 0,06g du composé de l'EXEMPLE 33 dans 0,5 ml de pyridine. On agite à 0°C pendant, 3 heures, ajoute 5 ml, d'eau et extrait à l'àcétate d'éthyle. On sèche la phase organique sur Na₂SO₄, évapore sous pression reduite, chromatographie sur une colonne de gel de silice en éluant avec un mélange cyclohexane/acétate d'éthyle 85/15 (v/v) pour isoler le produit attendu; F = 79°C
b) On ajoute à 0,04 g du composé précédent en solution dans 0,5 ml de diméthylformamide et 1 ml d'acétonitrile, 0,02 g de morpholine et 0,01 g d'iodure de sodium. Le mélange réactionnel est chauffé à 75°C pendant 16 heures. A 10°C, on ajoute. 5 ml d'eau, extrait à l'acétate d'éthyle, évapore les solvants, chromatographie sur colonne de gel de silice en éluant avec un mélange dichlorométhane/méthanol 98/2 (v/v). On traite avec une solution d'HCl dans l'éther diéthylique, filtre et séche à 50°C sous pression réduite pour isoler le composé sous forme de chlorhydrate ; F = 157°C (1HCl, 1H₂O).

### EXEMPLE 37

### 5-Chloro-1-[4-(N-tert-butylcarbamoyl-2-méthoxy)benzénesulfonyl]-3-spiro-(4-(2-tert-butoxycarbonylaminoéthyl)cyclohexane]indolin-2-one (Isomére le moins polaire)

(I): R₁ = 5-Cl; R₂=H; R₃=2-OCH₃; W=SO₂; R₄=4-CONHC(CH₃)₃;
Y₁ = -(CH₂)₂NHCOOC(CH₃₎₃ ;Y₂ = H

A -40°C, on ajoute à 0,10 g du composé (I'6) dans 2,5 ml de trétrahydrofurane et 0,08 g de chlorure de (2-méthoxy-4-N-*tert*-butylcarbamoyl)benzènesulfonyle, 0,03 g de *tert*-butylate de potassium. On agite à 20°C pendant 2 heures puis on ajoute 5 ml d'eau et extrait à l'acétate d'éthyle. On sèche sur sulfate de sodium, évapore les solvants. On isole le composé attendu par chromatographie sur une colonne de gel de silice en éluant avec un mélange cyclohexane/acétate d'éthyle 90/10 (v/v) ; F = 102°C.

### EXEMPLE 38

### 5-Chloro-1-[4-(N-tert -butylcarbamoyl-2-méthoxy)benzénesulfonyl]-3-spiro-[4-(2-tert-butoxycarbonylaminoéthyl)cyclohexane]indolin-2-one (Isomère le plus polaire)

(I) : R₁= 5-OC₂H₅ ; R₂ = H ; R₃ = 2-OCH₃ ; W = SO₂ ; R₄ = 4-CONHC(CH₃)₃ ;
Y₁= -(CH₂)₂NHCOOC(CH₃)₃ ; Y₂ = H

Préparé de la même manière que dans l'EXEMPLE 37 à partir du composé (I'7) ; F =107°C (1 cyclohexane).

### EXEMPLE 39

### 5-Chloro-3-spiro-[4-(2-aminoéthyl)cyclohexane]-1-[4-N-tert-butylcarbamoyl) benzènesulfonyl]indolin-2-one (Isomère le plus polaire)

(I) : R₁ = 5-Cl ; R₂ = H ; R₃ = 2-OCH₃ ; W =SO₂ : R₄ = 4-CONHC(CH₃)₃;
Y₁=-(CH₂)₂NH₂ ; Y₂ = H

A 0,025 g du composé de l'EXEMPLE 37, dans 0,25ml d'acétate d'éthyle, on ajoute. à 0°C sous atmosphère inerte 0,25 ml d'une, solution de chlorure d'hydrogène dans l'acétate d'éthyle saturée à 15°C. On agite à 20°C pendant 3 heures, on évapore le solvant sous pression réduite et reprend le résidu à l"éther diéthylique. On obtient le produit attendu après filtration et séchage à 50°C sous pression réduite pendant 5 heures; F = 169°C (HCI, H₂O).

### EXEMPLE 40

### 5-Chloro-3-spiro-[4-(2-aminoéthyl)cyclohexane]-1-[4-N-tert-butylcarbamoyl)benzénesulfonyl]indolin-2-one (Isomère le moins polaire)

(I) : R₁ = 5-Cl; R₂ = H ; R₃ = 2-OCH₃ ; W = SO₂ ; R₄ = 4-CONHC(CH₃)₃ ;
Y₁ = -(CH₂)₂NH₂ ; Y₂ = H

Préparé à partir du composé de l'EXEMPLE 38 selon le méme mode opératoire que dans l'EXEMPLE 39. F = 193 ° C (HCI).

### EXEMPLE 42

### 5-Chloro-1[(2-méthoxy-4-tert-butylcarbamoyl)benzènesulfonyl]-3-spiro-[(4-tert-butoxycarbonylaminométhyl)cyclohexane]indolin-2-one

(I) : R₁ = 5-Cl ; R₂= H ; R₃ = 2-OCH₃; W = SO₂ ; R₄= CONHC(CH₃)₃ ;
Y₁ =(CH₂)NHCOOC(CH₃)₃; Y₂=H

A une solution de 0,09 g du composé (I'8) et de 0,08 g de chlorure de (4-*tert*-butylcarbamoyl-2-méthoxy)benzènesulfonyle dans 2,5 ml de trètrahydrofurane, on ajoute à -50°C, 0,03 g de *tert*-butylate de potassium. A 20°C, on ajoute 4 ml d'eau, extrait à l'acétate d'étyle. sèche sûr sulfate de sodium, évapore les solvants sous pression rèduite. On isole le produit attendu (mélange d'isomères) après une chromatographie sur une colonne de gel de silice en éluant avec du dichlorométhane; F = 95°C.

### EXEMPLE 43

### 5-Chloro-3-spiro-(4-tert-butoxycarbonylaminométhylcyclohexane)-1-[(2,4-diméthoxy)benzènesulfonyl]indolin-2-one

(I): R₁ = 5-Cl; R₂ = H ; R₃= 2-OCH₃ ; W = SO₂ ; R₄ = 4-OCH₃;
Y₁ = (CH₂)NHCOOC(CH₃)₃; Y₂ = H

Préparé selon le même mode opératoire que dans l'EXEMPLE 42 à partir du chlorure de (2,4-diméthoxy)benzènesulfonyle.

1H RMN:7,9 (m,1H); 7,6 (m, 2H) ; 7,4 (m, 0,3H) ; 7,3 (m, 0,7H) ; 6,8-6,6 (m, 2H) ; 3,8 (s, 3H) ; 3,5 (s, 3H) ; 3,0 (m, 0;6H) ; 2,8 (m, 1,4H) ; 1,9-1,4 (m, 9H); 1,4 (s, 6,3H) ; 1,3 (s, 2,7H)

### EXEMPLE 44

### 5-Chloro-3-spiro-(4-aminométhylcyclohexane)-1-[(2,4-diméthoxy) benzènesulfonyl] indolin-2-one

(I) : R₁=5-Cl;R₂ = H ; R₃ = 2-OCH₃ ; W = SO₂ ; R₄ = 2-OCH₃;
Y₁ = (CH₂)NH₂ ; Y₂=H

Préparé à partir du composé de l'EXEMPLE 43 selon le même mode opératoire que dans l'EXEMPLE 39 ; F = 195°C (HCl).

## Revendications

1. Composés de formule (I) : 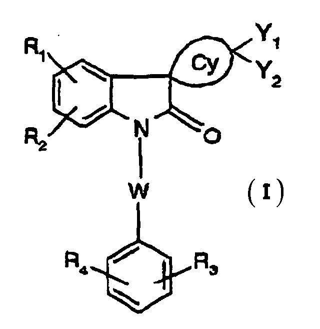 dans laquelle :
- R₁ est en position 5 de l'indolin-2-one et représente un hydrogéne ; un hydroxyle; un halogéne ; un (C₁-C₇)alkyle; un (C₁-C₇)polyfluoroalkyle; un (C₁-C₇)alcoxy; un (C₁-C₇)alkylthio; un (C₁-C₇)polyfluoroalcoxy; un (C₃-C₇)cycloalkyloxy; un (C₃-C₇) cycloalkylthio ; un cycloalkylméthoxy ou un cycloalkylméthylthio dans lesquels le cycloalkyle est en C₃-C₇; un phénoxy; un benzyloxy; un nitro; un cyano;
- R₂ représente l'hydrogène;
- R₃ et R₄ indépendamment l'un de l'autre substituent une ou plusieurs fois le groupe phényle et représentent chacun indépendamment un hydrogène : un halogène; un (C₁-C₇)alkyle ; un (C₂-C₇)alcényle ; un (C₁-C₇)polyhalogénoalkyle; un phényle ou un benzyle;un cyano ; un nitro ; un groupe -NR₅R₆ ; un hydroxyamino ; un hydroxyle; un groupe OR₇ ; un groupe SR₇ ; un groupe -COOR₈ ; un groupe -CONR₉R₁₀ ; un groupe -CSNR₉R₁₀, l'un au moins des radicaux R₃ et R₄ étant dlfférent de l'hydrogène ;
- R₅ et R₆ représentent chacun indépendamment un hydrogène ; un (C₁-C₇)alkyle ; un (C₂-C₇)alcényle ; un phényle ; un benzyle ; un (C₁-C₇)alkylcarbonyle ; un (C₁-C₇)alkylthiocarbonyle ; un (C₃-C₇)cycloalkycarbonyle ; un (C₃-C₇) cycloalkylthiocarbonyle ; un benzoyle; un thiénylcarbonyle ; un furylcarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀ ou bien R₅ et R₆ constituent avec l'atome d'azote auquel ils sont liés un groupe hétérocyclique choisi parmi les groupes pyrrolidine, pyrroline, pyrrole, indoline, indole, pipéridine;
ou bien R₅ constitue avec l'atome d'azote auquel il est lié et l'atome de carbone adjacent du phényle, un hétérocycle choisi parmi indole, indoline et tétrahydroquinoline et alors R₆ représente un hydrogène ; un (C₁-C₇)alkyle; un benzyle ; un (C₁-C₇)alkylcarbonyle ; un (C₁-C₇)alkylthiocarbonyle ; un (C₃-C₇)cycloalkylcarbonyle ; un (Ç₃-C₇)cycloalkylthiocarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀;
- R₇ représente un (C₁-C₇)alkyle ; un (C₂-C₇)alcényle ; un phényle ; un benzyle ; un (C₃-C₇)cycloalkyle ; un (C₁-C₇)polyfluoroalkyle ; un formyle ; un (C₁-C₇) alkylcarbonyle ; un benzoyle ; un benzylcarbonyle ;
- R₈ représente un hydrogène, un (C₁-C₇)alkyle; un phényle ; un benzyle ;
- R₉ et R₁₀ représentent chacun indépendamment l'hydrogèn un (C₁-C₇)alkyle; un (C₁-C₇)polyfluoroalkyle ; un (C₂-C₇)alcényle ; un (C₃-C₇)cycloalkyle éventuellement substitué par un groupe hydroxy(C₁-C₄)alkyle ; un pyridyle ; un phényle ; un thiényle ; un furyle ; ou bien R₉ et R₁₀ constituent avec l'atome d'azote auquel ils sont liés un groupe hétérocyclique choisi parmi les groupes pyrrolidine, pipéridine ou pipérazine non substitués ou substitués par un ou plusieurs groupe(s) (C₁-C₄)alkyle(s) ; ou un groupe (C₄-C₇)azacycloalkyle ;
- W représente un groupe -SO₂- ;
- Cy constitue, avec le carbone auquel il est lié, un cycle hydrocarboné non aromatique en C₅-C₁₂, saturé ou insaturé, éventuellement condensé ou substitué par un ou plusieurs groupe(s) (C₁-C₇)alkyle(s), lesdits groupes pouvant substituer une ou plusieurs fois le même atome de carbone, ou par un spirocycloalkyle en C₃-C₆;
- Y₁ et Y₂ substituent le même atome de carbone de Cy et
- Y₁ représente
(i) - un groupe (C₀-C₄)alkylène -T-Z ; ou
(ii) - un groupe (C₀-C₃)alkylène -NR₁₆-T-Z dans lequel R₁₆ représente un atome d'hydrogène ou un (C₁-C₃)alkyle;
- Y₂ représente un atome d'hydrogène ou un groupe hydroxyle ou bien forme avec Y₁ un groupe (C₁-C₄)alkylidène-T-Z ;
- T représente un (C₁-C₄)alkylène ou une liaison directs ;
- Z représente un groupe -NR₁₁R₁₂ ; un groupe -COOR₁₁ ; un groupe -CONR₁₁ R₁₂ ; étant entendu que lorsque Y₁ est tel que défini dans le cas (ii) et lorsque T représente un groupe méthylène ou une liaison directe, alors Z ne peut pas être -NR₁₁R₁₂;
- R₁₁ et R₁₂ représentent chacun indépendamment l'hydrog ou un (C₁-C₇)alkyle, lesdits groupes pouvant éventuellement être mono- ou polysubstitués par R₁₃;
ou bien R₁₁ et R₁₂ constituent éventuellement avec l'atome d'azote auquel ils sont liés un hétérocycle choisi parmi les hétérocycles pipéridine et morpholine, éventuellement mono ou polysubstitué par R₁₃;
- R₁₃ représente un benzyloxy ; un groupe NR₁₄R₁₅ dans lequel R₁₄ et R₁₅ représentent chacun indépendamment l'hydrogène ou un (C₁-C₄)alkyle;
ainsi que leurs sels, solvates ou hydrates.

2. Composés selon la revendication 1 dans lesquels Cy représente un cyclohexyle ; Y₁ et Y₂ substituent la position 4 du cyclohexyle; R₁ représente un éthoxy ; R₃ et R₄ étant tels que définis pour (I), ainsi que leurs sels, hydrates ou solvates.

3. Procédé de préparation d'un composé selon la revendication 1 ou 2 **caractérisé en ce que**:
1) on fait réagir un composé (I') de formule: 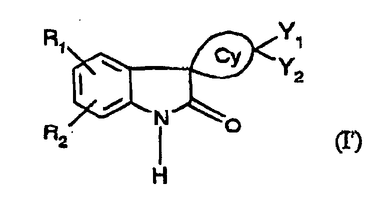 dans lequel R₁, R₂, Cy, Y₁ et Y₂ sont tels que définis pour (I) avec un composé de formule (2): 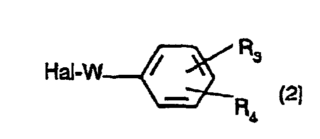 dans laquelle W, R₃ et R₄ sont tels que définis pour (I) et Hal représente un atome d'halogène, en présence d'un hydrure métallique ou d'un alcoolate alcalin à des températures comprises entre -40° et 25°C, dans un solvant anhydre ;
2) ou bien on fait réagir un réactif nucléophile tels que des thiols, amines, ou carbanions sur les dérivés carbonylés de formule (IIA): 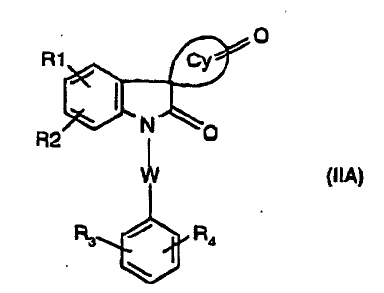
dans laquelle R₁, R₂, R₃, R₄, W et Cy sont tels que définis pour (I) pour obtenir directement les composés (I).

4. Composition pharmaceutique contenant, en tant que principe actif, un composé de formule (I) selon la revendication 1 ou un de ses sels, hydrates ou solvates pharmaceutiquement acceptables.

5. Composition pharmaceutique contenant en tant principe actif, un composé selon la revendication 2 ou un de ses sels, hydrates ou solvates pharmaceutiquement acceptables.

6. Composition pharmaceutique selon la revendication 4 ou 5 contenant également un autre principe actif.

7. Composition pharmaceutique selon la revendication 6 **caractérisée en ce que** l'autre principe actif est un antagoniste spécifique du récepteur de l'angiotensine II.

8. Composition pharmaceutique selon la revendication 7 **caractérisée en ce que** l'antagoniste spécifique du récepteur de l'angiotensine II est l'irbésartan.

## Patentansprüche

1. Verbindungen der Formel (I): 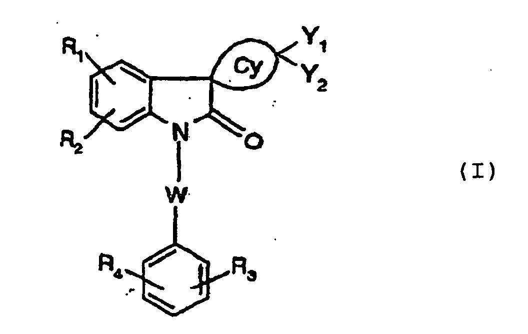 worin;
- R₁ in Position 5 von Indolin-2-on steht und Wasserstoff; ein Hydroxyl; ein Halogen; ein (C₁-C₇)-Alkyl; ein (C₁-C₇) -Polyfluoralkyl; ein (C₁-C₇) -Alkoxy; ein (C₁-C₇) -Alkylthio; ein (C₁-C₇) -Polyfluoralkoxy; ein (C₃-C₇) -Cycloalkyloxy; ein (C₃-C₇) -Cycloalkylthio; ein Cycloalkylmethoxy oder ein Cycloalkylmethylthio, worin Cycloalkylthio (C₃-C₇) ist; ein Phenoxy; ein Benzyloxy; ein Nitro; ein Cyano darstellt ;
- R₂ Wasserstoff darstellt;
- R₃ und R₄ unabhängig voneinander ein- oder mehrmals die Phenylgruppe substituieren und jeweils unabhängig Wasserstoff; ein Halogen; ein (C₁-C₇) -Alkyl; ein (C₂-C₇)-Alkenyl; ein (C₁-C₇)-Polyhalogenalkyl; ein Phenyl oder ein Benzyl; ein Cyano; ein Nitro; eine Gruppe -NR₅R₆; ein Hydroxyamino; ein Hydroxyl; eine Gruppe OR₇; eine Gruppe SR₇; eine Gruppe -COOR₈; eine Gruppe -CONR₉R₁₀; eine Gruppe -CSNR₉R₁₀ darstellen, wobei mindestens einer der Reste R₃ und R₄ nicht Wasserstoff ist;
- R₅ und R₆ jeweils unabhängig Wasserstoff; ein (C₁-C₇)-Alkyl; ein (C₂-C₇)-Alkenyl; ein Phenyl; ein Benzyl; ein (C₁-C₇)-Alkylcarbonyl; ein (C₁-C₇)-Alkylthiocarbonyl; ein (C₃-C₇) -Cycloalkylcarbonyl; ein (C₃-C₇)-Cycloalkylthiocarbonyl; ein Benzoyl; ein Thienylcarbonyl; ein Furylcarbonyl; ein (C₁-C₇) -Alkoxycarbonyl; ein Phenoxycarbonyl; ein Benzyloxycarbonyl; ein Carbamoyl oder ein Thiocarbamoyl, gegebenenfalls substituiert durch R₉ und R₁₀, darstellen, oder aber R₅ und R₆ mit dem Stickstoffatom, an das sie gebunden sind, eine heterocyclische Gruppe ausgewählt aus den Gruppen Pyrrolidin, Pyrrolin, Pyrrol, Indolin, Indol, Piperidin, darstellen;
oder aber R₅ mit dem Stickstoffatom, an das es gebunden ist, und dem Kohlenstoffatom neben dem Phenyl einen Heterozyklus ausgewählt aus Indol, Indolin und Tetrahydrochinolin darstellt und dann R₆ wasserstoff; ein (C₁-C₇)-Alkyl; ein Benzyl; ein (C₁-C₇)-Alkylcarbonyl; ein (C₁-C₇)-Alkylthiocarbonyl; ein (C₃-C₇)-Cycloalkylcarbonyl; ein (C₃-C₇)-Cycloalkylthiocarbonyl; ein (C₁-C₇)-Alkyloxycarbonyl; ein Phenoxycarbonyl; ein Benzyloxycarbonyl; ein Carbamoyl oder ein Thiocarbamoyl, gegebenenfalls substituiert durch R₉ and R₁₀, darstellt;
- R₇ ein (C₁-C₇)-Alkyl; ein (C₂-C₇)-Alkenyl; ein Phenyl; ein Benzyl ; ein (C₃-C₇)-Cycloalkyl; ein (C₁-C₇)-Polyfluoralkyl; ein Formyl; ein (C₁-C₇)-Alkylcarbonyl; ein Benzoyl; ein Benzylcarbonyl darstellt;
- R₈ Wasserstoff, ein (C₁-C₇)-Alkyl; ein Phenyl; ein Benzyl darstellt;
- R₉ and R₁₀ jeweils unabhängig Wasserstoff; ein (C₁-C₇,)-Alkyl; ein (C₁-C₇)-Polyfluoralkyl; ein (C₂-C₇)-Alkenyl; ein (C₃-C₇)-Cycloalkyl, gegebenenfalls substituiert durch eine Hydroxy-(C₁-C₄)-Alkylgruppe; ein Pyridyl; ein Phenyl; ein Thienyl; ein Furyl darstellen; oder aber R₉ und R₁₀ mit dem Stickstoffatom, an das sie gebunden sind, eine heterocyclische Gruppe ausgewählt aus den Gruppen Pyrrolidin, Piperidin oder Piperazin, gegebenenfalls substituiert durch eine oder mehrere (C₁-C₇)-Alkylgruppe(n); oder ein (C₄-C₇) -Azacycloalkyl darstellen;
- W eine Gruppe -SO₂- darstellt;
- Cy mit dem Kohlenstoff, an den es gebunden ist, einen gesättigten oder ungesättigten nicht aromatischen C₅-C₁₂-Kohlenstoffring darstellt, gegebenenfalls kondensiert oder substituiert durch eine oder mehrere (C₁-C₇)-Alkylgruppe(n), wobei die Gruppen dasselbe Kohlenstoffatom ein- oder mehrmals substituieren können, oder durch ein C₃-C₆-Spirocycloalkyl;
- Y₁ und Y₂ dasselbe Kohlenstoffatom von Cy substituieren und
- Y₁ folgendes darstellt:
(i) - eine Gruppe (C₀-C₄)-Alkylen-T-Z; oder
(ii) - eine Gruppe (C₀-C₃)-Alkylen-NR₁₆-T-Z, worin R₁₆ ein Wasserstoffatom oder ein (C₁-C₃)-Alkyl bedeutet;
- Y₂ ein Wasserstoffatom oder eine Hydroxylgruppe darstellt oder aber mit Y₁ eine Gruppe (C₁-C₄) -Alkyliden-T-Z bildet;
- T (C₁-C₄)-Alkylen oder eine direkte Bindung darstellt;
- Z eine Gruppe -NR₁₁R₁₂; eine Gruppe -COOR₁₁; eine Gruppe -CONR₁₁R₁₂ darstellt, mit der Maßgabe, dass, wenn Y₁ wie im Fall (ii) definiert ist und T eine Methylengruppe oder eine direkte Bindung darstellt, Z dann nicht -NR₁₁R₁₂ sein kann;
- R₁₁ and R₁₂ jeweils unabhängig Wasserstoff oder ein (C₁-C₇) -Alkyl darstellen, welche Gruppen gegebenenfalls durch R₁₃ mono- oder polysubstituiert sein können;
oder aber R₁₁ and R₁₂ gegebenenfalls mit dem Stickstoffatom, an das sie gebunden sind, einen Heterozyklus bilden, ausgewählt aus den Heterozyklen Piperidin und Morpholin, gegebenenfalls durch R₁₃ mono- oder polysubstituiert;
- R₁₃ ein Benzyloxy; eine Gruppe NR₁₄R₁₅ darstellt, worin R₁₄ and R₁₅ jeweils unabhängig Wasserstoff oder ein (C₁-C₄)-Alkyl darstellen; sowie die Salze, Solvate oder Hydrate derselben.

2. Verbindungen nach Anspruch 1, worin Cy ein Cyclohexyl darstellt; Y₁ und Y₂ die Position 4 des Cyclohexyls substituieren; R₁ ein Ethoxy darstellt; R₃ und R₄ wie für (I) definiert sind, sowie die Salze, Hydrate oder Solvate derselben.

3. Verfahren zur Herstellung einer Verbindung nach Anspruch 1 oder 2, **dadurch gekennzeichnet, dass**:
1) eine Verbindung (I') der Formel: 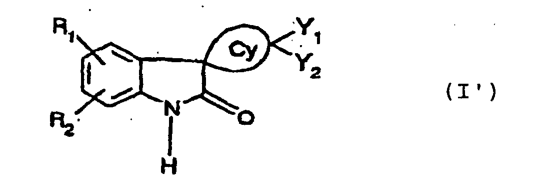 worin R₁, R₂, Cy, Y₁ und Y₂ wie für (I) definiert sind, mit einer Verbindung der Formel (2): 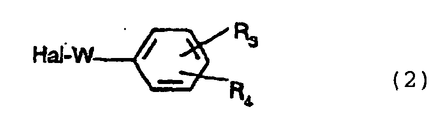 worin W, R₃ und R₄ wie für (I) definiert sind und Hal ein Halogenatom darstellt, in Anwesenheit eines Metallhydrids oder eines Alkalialkoholats bei Temperaturen zwischen -40° und 25°C in einem wasserfreien Lösungsmittel zur Umsetzung gebracht wird;
2) oder aber ein nukleophiles Reagens wie Thiole, Amine oder Carbanionen mit den Carbonylderivaten der Formel (IIA): 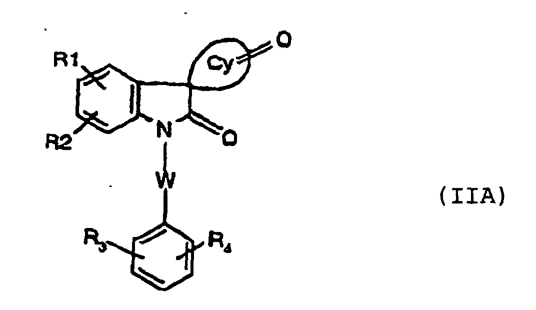
worin R₁, R₂, R₃, R₄, W und Cy wie für (I) definiert sind, zur Umsetzung gebracht wird, um die Verbindungen (I) direkt zu erhalten.

4. Pharmazeutische Zusammensetzung, die als Wirkstoff eine Verbindung der Formel (I) nach Anspruch 1 oder eines ihrer pharmazeutisch annehmbaren Salze, Hydrate oder Solvate enthält.

5. Pharmazeutische Zusammensetzung, die als Wirkstoff eine Verbindung nach Anspruch 2 oder eines ihrer pharmazeutisch annehmbaren Salze, Hydrate oder Solvate enthält.

6. Pharmazeutische Zusammensetzung nach Anspruch 4 oder 5, die auch einen anderen Wirkstoff enthält.

7. Pharmazeutische Zusammensetzung nach Anspruch 6, **dadurch gekennzeichnet, dass** der andere Wirkstoff ein spezifischer Antagonist des Angiotensin-II-Rezeptors ist.

8. Pharmazeutische Zusammensetzung nach Anspruch 7, **dadurch gekennzeichnet, dass** der spezifische Antagonist des Angiotensin-II-Rezeptors Irbesartan ist.

## Claims

1. Compounds with formula (I): 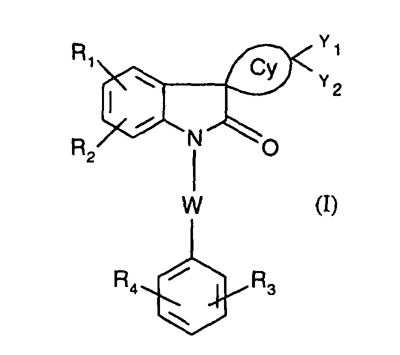 where
• R₁ is in the 5 position of the indolin-2-one and represents a hydrogen; a hydroxyl; a halogen; a (C₁-C₇)alkyl; a (C₁-C₇)polyfluoroalkyl; a (C₁-C₇)alkoxy; a (C₁-C₇)alkylthio; a (C₁-C₇)polyfluoroalkoxy; a (C₃-C₇)cycloalkyloxy; a (C₃-C₇)cycloalkylthio; a cycloalkylmethoxy or a cycloalkylmethylthio in which the cycloalkyl is C₃-C₇; a phenoxy; a benzyloxy; a nitro; a cyano;
• R₂ represents hydrogen;
• R₃ and R₄, independently of each other, substitute the phenyl group one or more times and each independently represent hydrogen; a halogen; a (C₁-C₇)alkyl; a (C₂-C₇)alkenyl; a (C₁-C₇)polyhalogenoalkyl; a phenyl or a benzyl; a cyano; a nitro; a -NR₅R₆ group; a hydroxyamino; a hydroxyl; a OR₇ group; a SR₇ group; a -COOR₈ group; a -CONR₉R₁₀ group; a -CSNR₉R₁₀ group, at least one of radicals R₃ and R₄ being other than hydrogen;
• R₅ and R₆ each independently represent a hydrogen; a (C₁-C₇)alkyl; a (C₂-C₇)alkenyl; a phenyl; a benzyl; a (C₁-C₇)alkylcarbonyl; a (C₁-C₇)alkylthiocarbonyl; a (C₃-C₇)cycloalkylcarbonyl; a (C₃-C₇)cycloalkylthiocarbonyl; a benzoyl; a thienylcarbonyl; a furylcarbonyl; a (C₁-C₇)alkyloxycarbonyl; a phenoxycarbonyl; a benzyloxycarbonyl; a carbamoyl or a thiocarbamoyl unsubstituted or substituted by R₉ and R₁₀, or R₅ and R₆ together with the nitrogen to which they are bonded constitute a heterocyclic group selected from pyrrolidine, pyrroline, pyrrole, indoline, indole, piperidine groups;
• or R₅ together with the nitrogen atom to which it is bonded and the adjacent carbon atom of the phenyl group constitutes a heterocycle selected from indole, indoline and tetrahydroquinoline, and then R₆ represents a hydrogen; a (C₁-C₇)alkyl; a benzyl; a (C₁-C₇)alkylcarbonyl; a (C₁-C₇)alkylthiocarbonyl; a (C₃-C₇)cycloalkylcarbonyl; (a C₃-C₇)cycloalkylthiocarbonyl; a (C₁-C₇)alkyloxycarbonyl; a phenoxycarbonyl; a benzyloxycarbonyl; a carbamoyl or a thiocarbamoyl group unsubstituted or substituted by R₉ and R₁₀;
• R₇ represents a (C₁-C₇)alkyl; a (C₂-C₇)alkenyl; a phenyl; a benzyl; a (C₃-C₇)cycloalkyl; a (C₁-C₇)polyfluoroalkyl; a formyl; a (C₁-C₇) alkylcarbonyl; a benzoyl; a benzylcarbonyl;
• R₈ represents a hydrogen; a (C₁-C₇)alkyl; a phenyl; a benzyl;
• R₉ and R₁₀ each independently represent hydrogen; a (C₁-C₇)alkyl; a (C₁-C₇)polyfluoroalkyl; a (C₂-C₇)alkenyl; a (C₃-C₇)cycloalkyl optionally substituted by a hydroxy(C₁-C₄)alkyl group; a pyridyl; a phenyl; a thienyl; a furyl; or R₉ and R₁₀ together with the nitrogen atom to which they are bonded constitute a heterocyclic group selected from pyrrolidine, piperidine or piperazine groups not substituted or substituted by one or more (C₁-C₄)alkyl group(s); or a (C₄-C₇)azacycloalkyl group;
• W represents a -SO₂- group;
• Cy constitutes, together with the carbon atom to which it is bonded, a non aromatic, saturated or unsaturated C₅-C₁₂ hydrocarbon cycle, optionally condensed or substituted by one or more (C₁-C₇)alkyl group(s), said groups possibly substituting the same carbon atom one or more times, or by a C₃-C₆ spirocycloalkyl group;
• Y₁ and Y₂ substitute the same carbon atom of Cy, and
• Y₁ represents either
(i) - a (C₀-C₄)alkylene -T-Z group,
(ii) - a (C₀-C₃)alkylene -NR₁₆-T-Z group in which R₁₆ represents a hydrogen atom or a (C₁-C₃)alkyl ;
• Y₂ represents a hydrogen atom or a hydroxyl group or forms with Y₁ a (C₁-C₄)alkylidene-T-Z- group;
• T represents a (C₁-C₄)alkylene or a direct bond;
• Z represents a -NR₁₁R₁₂ group; a -COOR₁₁ group; a -CONR₁₁R₁₂ group, it being understood that:
◆ when Y₁ is as defined in case (ii) and when T represents a methylene group or a direct bond, Z cannot be a -NR₁₁R₁₂;
• R₁₁ and R₁₂ each independently represent hydrogen or a (C₁-C₇)alkyl; said groups optionally being mono or polysubstituted by R₁₃;
or R₁₁ and R₁₂, together with the nitrogen atom to which they are bonded, optionally constitute a heterocycle selected from the heterocycles : piperidine and morpholine, optionally mono- or polysubstituted by R₁₃;
R₁₃ represents a benzyloxy; a NR₁₄R₁₅ group where R₁₄ and R₁₅ each independently represent hydrogen or a (C₁-C₄)alkyl;
and their salts, solvates and hydrates.

2. Compounds according to claim 1, in which Cy represents a cyclohexyl, Y₁ and Y₂ substitute the 4 position of the cyclohexyl ; R₁ represents ethoxy ; R₃ and R₄ are as defined for (I), and their salts, hydrates or solvates.

3. A process for preparing a compound with formula (I) according to claim 1 or 2, **characterized in that**:
1 ) a compound (I') with formula: 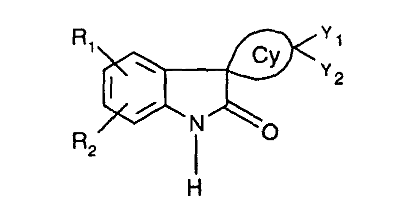 where R₁, R₂, Cy, Y₁ and Y₂ are as defined for (I) is reacted with a compound with formula (2): 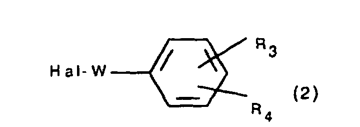 where W, R₃ and R₄ are as defined for (I) and Hal represents a halogen atom, is reacted in the presence of a metallic hydride or an alkaline alcoholate at temperatures comprised between -40°C and 25°C, in an anhydrous solvent;
2) or a nucleophilic reagent such as thiols, amines or carbanions, is reacted with carbonyl derivatives (IIA): 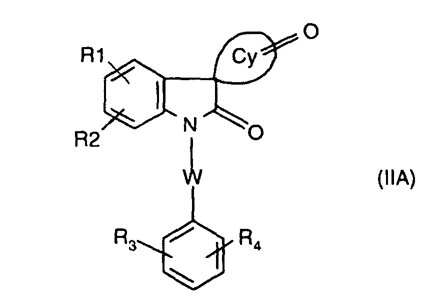
in which R₁, R₂, R₃, R₄, W and Cy are as defined for (I) to directly obtain compounds (I).

4. A pharmaceutical composition containing, as an active principle, a compound with formula (I) according to claim 1 or one of its pharmaceutically acceptable salts, hydrates or solvates.

5. A pharmaceutical composition containing, as an active principle, a compound with formula (I) according to claim 2 or one of its pharmaceutically acceptable salts, hydrates or solvates.

6. A pharmaceutical composition according to claim 4 or 5, also containing a further active principle.

7. A pharmaceutical composition according to claim 6, **characterized in that** the further active principle is a specific antagonist for the angiotensin II receptor.

8. A pharmaceutical composition according to claim 7, **characterized in that** the specific antagonist for the angiotensin II receptor is irbesartan.
